# EUROPEAN PATENT SPECIFICATION

(11) **EP 2 279 013 B1**
(45) Date of publication and mention of the grant of the patent: **05.08.2015**
(21) Application number: 09735127.4
(22) Date of filing: 24.04.2009
(51) Int. Cl.: A61L 17/10, A61B 17/064, A61L 17/00, D06M 11/83, D06M 15/00, D01F 6/00, A61B 17/00, A61B 17/06

(54) **SHAPE-MEMORY SELF-RETAINING SUTURES, METHODS OF MANUFACTURE, AND METHODS OF USE**
SELBSTHALTENDE FORMGEDÄCHTNISNAHT, HERSTELLUNGSVERFAHREN UND ANWENDUNGSVERFAHREN
SUTURES AUTO-RÉTENTIVES À MÉMOIRE DE FORME, PROCÉDÉS DE FABRICATION, ET PROCÉDÉS D UTILISATION

(30) Priority: 24.04.2008 US 47682 P
(43) Date of publication of application: 02.02.2011
(73) Proprietor: Ethicon LLC, Guaynabo 00969 (PR)
(72) Inventor: GORALTCHOUK, Alexei, Goleta CA 93117 (US); LAI, John, Richmond V6V 2T3 (CA); HERRMANN, Robert, A, Vancouver V6B 1X9 (CA)
(74) Representative: Small, Gary James
(86) International application number: PCT/US2009/041685
(87) International publication number: WO 2009/132284

(56) References cited:
- WO-A2-03/017850
- WO-A2-2004/030704
- US-A1- 2004 015 187
- US-A1- 2005 267 532
- US-A1- 2007 005 109
- US-A1- 2007 005 110
- US-A1- 2007 005 110
- US-A1- 2007 224 237

## Description

### FIELD OF INVENTION

The present invention relates generally to self-retaining systems for surgical procedures, methods of manufacturing self-retaining systems for surgical procedures, and uses thereof.

### BACKGROUND OF INVENTION

Wound closure devices such as sutures, staples and tacks have been widely used in superficial and deep surgical procedures in humans and animals for closing wounds, repairing traumatic injuries or defects, joining tissues together (bringing severed tissues into approximation, closing an anatomical space, affixing single or multiple tissue layers together, creating an anastomosis between two hollow/luminal structures, adjoining tissues, attaching or reattaching tissues to their proper anatomical location), attaching foreign elements to tissues (affixing medical implants, devices, prostheses and other functional or supportive devices), and for repositioning tissues to new anatomical locations (repairs, tissue elevations, tissue grafting and related procedures) to name but a few examples.

Sutures are often used as wound closure devices. Sutures typically consist of a filamentous suture thread attached to a needle with a sharp point. Suture threads can be made from a wide variety of materials including bioabsorbable (i.e., that break down completely in the body over time), or non-absorbable (permanent; non-degradable) materials. Absorbable sutures have been found to be particularly useful in situations where suture removal might jeopardize the repair or where the natural healing process renders the support provided by the suture material unnecessary after wound healing has been completed; as in, for example, completing an uncomplicated skin closure. Non-degradable (non-absorbable) sutures are used in wounds where healing may be expected to be protracted or where the suture material is needed to provide physical support to the wound for long periods of time; as in, for example, deep tissue repairs, high tension wounds, many orthopedic repairs and some types of surgical anastomosis. Also, a wide variety of surgical needles are available, and the shape, and size of the needle body and the configuration of the needle tip is typically selected based upon the needs of the particular application.

To use an ordinary suture, the suture needle is advanced through the desired tissue on one side of the wound and then through the adjacent side of the wound. The suture is then formed into a "loop" which is completed by tying a knot in the suture to hold the wound closed. Knot tying takes time and causes a range of complications, including, but not limited to (i) spitting (a condition where the suture, usually a knot, pushes through the skin after a subcutaneous closure), (ii) infection (bacteria are often able to attach and grow in the spaces created by a knot), (iii) bulk/mass (a significant amount of suture material left in a wound is the portion that comprises the knot), (iv) slippage (knots can slip or come untied), and (v) irritation (knots serve as a bulk "foreign body" in a wound). Suture loops associated with knot tying may lead to ischemia (knots can create tension points that can strangulate tissue and limit blood flow to the region) and increased risk of dehiscence or rupture at the surgical wound. Knot tying is also labor intensive and can comprise a significant percentage of the time spent closing a surgical wound. Additional operative procedure time is not only bad for the patient (complication rates rise with time spent under anesthesia), but it also adds to the overall cost of the operation (many surgical procedures are estimated to cost between $15 and $30 per minute of operating time).

Self-retaining sutures (including barbed sutures) differ from conventional sutures in that self-retaining sutures possess numerous tissue retainers (such as barbs) which anchor the self-retaining suture into the tissue following deployment and resist movement of the suture in a direction opposite to that in which the retainers face, thereby eliminating the need to tie knots to affix adjacent tissues together (a "knotless" closure). Knotless tissue-approximating devices having barbs have been previously described in, for example, U.S. Pat. No. 5,374,268, disclosing armed anchors having barb-like projections, while suture assemblies having barbed lateral members have been described in U.S. Pat. Nos. 5,584,859 and 6,264,675. Sutures having a plurality of barbs positioned along a greater portion of the suture are described in U.S. Pat No. 5,931,855, which discloses a unidirectional barbed suture, and U.S. Pat. No. 6,241,747, which discloses US 2007/005110 discloses a suture assembly comprising: a plurality of unbarbed filamentary elements intertwined with one another; at least one barbed filamentary element having a longitudinal axis and having plurality of barbs extending outwardly therefrom in a first direction less than 90 degrees from the longitudinal axis; wherein the at least one barbed filamentary element is intertwined along its length with the plurality of unbarbed filamentary elements the barbed filamentary element is comprised of a shape memory material. Methods and apparatus for forming barbs on sutures have been described in, for example, U.S. Pat. Nos. 6,848,152. Self-retaining systems for wound closure also result in better approximation of the wound edges, evenly distribute the tension along the length of the wound (reducing areas of tension that can break or lead to ischemia), decrease the bulk of suture material remaining in the wound (by eliminating knots) and reduce spitting (the extrusion of suture material - typically knots - through the surface of the skin. All of these features are thought to reduce scarring, improve cosmesis, and increase wound strength relative to wound closures using plain sutures or staples. Thus, self-retaining sutures, because such sutures avoid knot tying, allow patients to experience an improved clinical outcome, and also save time and costs associated with extended surgeries and follow-up treatments. It is noted that all patents, patent applications and patent publications identified throughout are incorporated herein by reference in their entirety.

The ability of self-retaining sutures to anchor and hold tissues in place even in the absence of tension applied to the suture by a knot is a feature that also provides superiority over plain sutures. When closing a wound that is under tension, this advantage manifests itself in several ways: (i) self-retaining sutures have a multiplicity of retainers which can dissipate tension along the entire length of the suture (providing hundreds of "anchor" points, this produces a superior cosmetic result and lessens the chance that the suture will "slip" or pull through) as opposed to knotted interrupted sutures which concentrate the tension at discrete points; (ii) complicated wound geometries can be closed (circles, arcs, jagged edges) in a uniform manner with more precision and accuracy than can be achieved with interrupted sutures; (iii) self-retaining sutures eliminate the need for a "third hand" which is often required for maintaining tension across the wound during traditional suturing and knot tying (to prevent "slippage" when tension is momentarily released during tying); (iv) self-retaining sutures are superior in procedures where knot tying is technically difficult, such as in deep wounds or laparoscopic/endoscopic procedures; and (v) self-retaining sutures can be used to approximate and hold the wound prior to definitive closure. As a result, self-retaining sutures provide easier handling in anatomically tight or deep places (such as the pelvis, abdomen and thorax) and make it easier to approximate tissues in laparoscopic/endoscopic and minimally invasive procedures; all without having to secure the closure via a knot. Greater accuracy allows self-retaining sutures to be used for more complex closures (such as those with diameter mismatches, larger defects or purse string suturing) than can be accomplished with plain sutures.

A self-retaining suture may be unidirectional, having one or more retainers oriented in one direction along the length of the suture thread; or bidirectional, typically having one or more retainers oriented in one direction along a portion of the thread, followed by one or more retainers oriented in another (often opposite) direction over a different portion of the thread (as described with barbed retainers in U.S. Pat. Nos. 5,931,855 and. 6,241,747). Although any number of sequential or intermittent configurations of retainers are possible, a common form of bidirectional self-retaining suture involves a needle at one end of a suture thread which has barbs having tips projecting "away" from the needle until the transition point (often the midpoint) of the suture is reached; at the transition point the configuration of barbs reverses itself about 180° (such that the barbs are now facing in the opposite direction) along the remaining length of the suture thread before attaching to a second needle at the opposite end (with the result that the barbs on this portion of the suture also have tips projecting "away" from the nearest needle). Projecting "away" from the needle means that the tip of the barb is further away from the needle and the portion of suture comprising the barb may be pulled more easily through tissue in the direction of the needle than in the opposite direction. Put another way, the barbs on both "halves" of a typical bidirectional self-retaining suture have tips that point towards the middle, with a transition segment (lacking barbs) interspersed between them, and with a needle attached to either end.

### BRIEF SUMMARY OF INVENTION

Despite the multitude of advantages of unidirectional and bidirectional self-retaining sutures, there remains a need to improve upon the design of the suture such that a variety of common limitations can be eliminated. Specifically, several problems common to existing self-retaining sutures can be addressed by the embodiments of this invention, including, but not limited to: (i) retainers or barbs that are fragile and break or too flexible and bend back, or do not stand proud due to an insufficient ability of the material to plastically deform and as such do not properly engage when deployed in tissue; (ii) inadequate "hold" provided by the retainers for some surgical procedures; resulting in retainers or barbs do not sufficiently anchor in the surrounding tissue and "pull through;" (iii) insufficient contact between the retainers and the surrounding tissue (often occurring when the thread diameter is too small relative to the diameter of the hole created by a larger needle; this limits the ability of the retainers to contact and "grip" the surrounding tissue); (iv) breakage of the self-retaining suture during tensioning and wound approximation; and (v) rotation and slippage of the retainers after deployment. Furthermore, the creation and or deployment of retainer features of self-retaining sutures may be difficult to achieve.

In accordance with the foregoing background and the limitations of the prior art, the present invention provides, shape-memory self-retaining sutures which have enhanced ability to anchor into the surrounding tissue, enhanced tissue holding capabilities, enhanced maximum load, and enhanced clinical performance.

In accordance with one aspect, there is provided a self-retaining suture comprising: a filament having a length; a plurality of retainers disposed on the filament, each retainer having a retainer base engaging the filament; and a first shape-memory material; wherein the self-retaining suture has a first form and a second form, and wherein a transition in the suture from the first form to the second form is effectuated by exposure to an electromagnetic stimulus; characterized in that the electromagnetic stimulus is selected from the group consisting of UV radiation, IR radiation, visible light, and laser light, and wherein the source of the stimulus is connectable to an end of the filament such that said stimulus travels through said filament. The self-retaining suture has a first form and a second form, and a transition in the suture from the first form to the second form is effectuated by a electromagnetic transition stimulus. In various embodiments, either the first form and/or the second form may include elevated retainers. Similarly, in various embodiments, either the first form and/or the second form may include an increase in the filament length. In certain embodiments, at least one of the filament and the retainers may include the shape-memory material. the transition stimulus is an electromagnetic radiation of a selected magnitude. In further embodiments, the suture may include a third form, wherein a transition in the suture from the second form to the third form is effectuated by a secondary transition stimulus. In some of the embodiments in which the suture includes a third form, the third form comprises elevated retainers and/or an increased filament length. In addition, in some of the embodiments including a third suture form, the secondary transition stimulus may be one or more of the following: a selected electric field, a selected magnetic field, electromagnetic radiation of a selected magnitude, a selected temperature, a selected pressure, a selected pH, a selected chemical environment, or a selected solvation environment. In certain embodiments, the shape-memory material may be selected from the class comprised of polymers, thermoplastics, metal alloys, hydrogels and ceramics. In particular embodiments, the shape-memory material may be a metal alloy, and in some of those embodiments, the metal alloy may be a nickel-titanium alloy. In some embodiments, the suture may comprise more than one material, and one of those materials may be the first shape-memory material. In some embodiments, the retainers may include at least in part the first shape-memory material. In some embodiments, in which the suture comprises more than one material, another of the materials may be a second shape-memory material. In some such embodiments, the retainers may include at least in part the second shape-memory material. In addition, in some embodiments suture may include a third material, and in some of these embodiments the third material may be a shape-memory material.

In further embodiments, suture may include a sheath layer and a core layer and at least one of these layers may include the first shape-memory material. In certain embodiments, the core layer may include braided core filaments, parallel core filaments, and/or segmented core filaments. In yet other embodiments, the suture may include a sheath layer, an intermediate layer, and a core layer. In certain of these embodiments, the core layer may include braided core filaments, parallel core filaments, and/or segmented core filaments.

In accordance with another aspect, there is provided a self-retaining suture system which includes at least one such suture.

The details of one or more aspects or embodiments are set forth in the description below. Other features, objects and advantages will be apparent from the description, the drawings, and the claims. In addition, the disclosures of all patents and patent applications referenced herein are incorporated by reference in their entirety.

### DESCRIPTION OF DRAWINGS

Features of the invention, its nature and various advantages will be apparent from the accompanying drawings and the following detailed description of various embodiments.
FIG. 1A is a view of a shape-memory self-retaining suture system in accordance with an embodiment of the present invention.
FIG. 1B is a view of a portion of the shape-memory self-retaining suture thread of the shape-memory self-retaining suture system of FIG. 1A.
FIGS. 1C and 1D are sectional views of the shape-memory self-retaining suture thread of FIGS. 1A and 1B illustrating the transition of a retainer from one shape to another shape in response to a transition stimulus.
FIG. 2A, is a flowchart of the process of making and programming a shape-memory self-retaining suture in accordance with one embodiment of the present invention.
FIGS. 2B to 2F show a shape-memory self-retaining suture during the steps of the process of 2A.
FIGS. 3A to 3C show the use of a shape-memory self-retaining suture to close a wound in accordance with an embodiment of the present invention.
FIG. 3D shows an alternative transition stimulus applied to the shape-memory self-retaining suture of FIGS. 3A-3C.
FIGS. 4A to 4G show the use of shape-memory self-retaining sutures to close wounds in accordance with embodiments of the present invention.
FIG. 4H shows the use of a shape-memory self-retaining suture to elevate tissue in accordance with an embodiment of the present invention.
FIGS. 5A to 5C show an alternative shape-memory self-retaining suture in accordance with an embodiment of the present invention.
FIGS. 6A to 6C show an alternative shape-memory self-retaining suture in accordance with an embodiment of the present invention.
FIGS. 6D to 6F show an alternative shape-memory self-retaining suture in accordance with an embodiment of the present invention.
FIGS. 7A to 7B show methods for manufacturing composite filaments suitable for making composite shape-memory self-retaining suture in accordance with embodiments of the present invention.
FIGS. 8A to 8H show various configurations of composite filaments suitable for creation of composite shape-memory self-retaining sutures in accordance with embodiments of the present invention.
FIGS. 9A to 9C show a composite shape-memory self-retaining suture in accordance with embodiments of the present invention.
FIGS. 10A to 10E show a self-retaining suture having shape-memory retainers in accordance with embodiments of the present invention.
FIGS. 10F to 10H show configurations of self-retaining suture systems having more than two arms in accordance with embodiments of the present invention.
FIGS. 11A to 11B show a shape-memory self-retaining suture having a therapeutic agent in accordance with an embodiment of the present invention.

### DESCRIPTION OF INVENTION

### Definitions

Definitions of certain terms that may be used hereinafter include the following.

"Self retaining system" refers to a self-retaining suture together with devices for deploying the suture into tissue. Such deployment devices include, without limitation, suture needles, cannulas and other deployment devices as well as sufficiently rigid and sharp ends on the suture itself to penetrate tissue.

"Self-retaining suture" refers to a suture that comprises features on the suture filament for engaging tissue without the need for a knot or suture anchor.

"Tissue retainer" (or simply "retainer") or "barb" refers to a physical feature of a suture filament which is adapted to mechanically engage tissue and resist movement of the suture in at least one axial directions. By way of example only, tissue retainer or retainers can include hooks, projections, barbs, darts, extensions, bulges, anchors, protuberances, spurs, bumps, points, cogs, tissue engagers, traction devices, surface roughness, surface irregularities, surface defects, edges, facets and the like. In certain configurations, tissue retainers are adapted to engage tissue to resist movement of the suture in a direction other than the direction in which the suture is deployed into the tissue by the surgeon, by being oriented to substantially face the deployment direction. In some embodiments the retainers lie flat when pulled in the deployment direction and open or "fan out" when pulled in a direction contrary to the deployment direction. As the tissue-penetrating end of each retainer faces away from the deployment direction when moving through tissue during deployment, the tissue retainers should not catch or grab tissue during this phase. Once the self-retaining suture has been deployed, a force exerted in another direction (often substantially opposite to the deployment direction) causes the retainers to be displaced from the deployment position (i.e. resting substantially along the suture body), forces the retainer ends to open (or "fan out") from the suture body in a manner that catches and penetrates into the surrounding tissue, and results in tissue being caught between the retainer and the suture body; thereby "anchoring" or affixing the self-retaining suture in place. In certain other embodiments, the tissue retainers may be configured to permit motion of the suture in one direction and resist movement of the suture in another direction without fanning out or deploying. In certain other configurations, the tissue retainer may be configured or combined with other tissue retainers to resist motion of the suture filament in both directions. Typically a suture having such retainers is deployed through a device such as a cannula which prevents contact between the retainers and the tissue until the suture is in the desired location.

"Retainer configurations" refers to configurations of tissue retainers and can include features such as size, shape, flexibility, surface characteristics, and so forth. These are sometimes also referred to as "barb configurations".

"Bidirectional suture" refers to a self-retaining suture having retainers oriented in one direction at one end and retainers oriented in the other direction at the other end. A bidirectional suture is typically armed with a needle at each end of the suture thread. Many bidirectional sutures have a transition segment located between the two barb orientations.

"Transition segment" refers to a retainer-free (barb-free) portion of a bidirectional suture located between a first set of retainers (barbs) oriented in one direction and a second set of retainers (barbs) oriented in another direction. The transition segment can be at about the midpoint of the self-retaining suture, or closer to one end of the self-retaining suture to form an asymmetrical self-retaining suture system.

"Suture thread" refers to the filamentary body component of the suture. The suture thread may be a monofilament, or comprise multiple filaments as in a braided suture. The suture thread may be made of any suitable biocompatible material, and may be further treated with any suitable biocompatible material, whether to enhance the sutures' strength, resilience, longevity, or other qualities, or to equip the sutures to fulfill additional functions besides joining tissues together, repositioning tissues, or attaching foreign elements to tissues.

"Monofilament suture" refers to a suture comprising a monofilamentary suture thread.

"Braided suture" refers to a suture comprising a multifilamentary suture thread. The filaments in such suture threads are typically braided, twisted, or woven together.

"Degradable suture" (also referred to as "biodegradable suture" or "absorbable suture") refers to a suture which, after introduction into a tissue is broken down and absorbed by the body. Typically, the degradation process is at least partially mediated by, or performed in, a biological system. "Degradation" refers to a chain scission process by which a polymer chain is cleaved into oligomers and monomers. Chain scission may occur through various mechanisms, including, for example, by chemical reaction (e.g., hydrolysis, oxidation/reduction, enzymatic mechanisms or a combination of these) or by a thermal or photolytic process. Polymer degradation may be characterized, for example, using gel permeation chromatography (GPC), which monitors the polymer molecular mass changes during erosion and breakdown. Degradable suture material may include polymers such as polyglycolic acid, copolymers of glycolide and lactide, copolymers of trimethylene carbonate and glycolide with diethylene glycol (e.g., MAXONTM, Tyco Healthcare Group), terpolymer composed of glycolide, trimethylene carbonate, and dioxanone (e.g., BIOSYNTM [glycolide (60%), trimethylene carbonate (26%), and dioxanone (14%)], Tyco Healthcare Group), copolymers of glycolide, caprolactone, trimethylene carbonate, and lactide (e.g., CAPROSYNTM, Tyco Healthcare Group). A dissolvable suture can also include partially deacetylated polyvinyl alcohol. Polymers suitable for use in degradable sutures can be linear polymers, branched polymers or multi-axial polymers. Examples of multi-axial polymers used in sutures are described in U.S. Patent Application Publication Nos. 2002/0161168, 2004/0024169, and 2004/0116620. Sutures made from degradable suture material lose tensile strength as the material degrades. Degradable sutures can be in either a braided multifilament form or a monofilament form.

"Non-degradable suture" (also referred to as "non-absorbable suture") refers to a suture comprising material that is not degraded by chain scission such as chemical reaction processes (e.g., hydrolysis, oxidation/reduction, enzymatic mechanisms or a combination of these) or by a thermal or photolytic process. Non-degradable suture material includes polyamide (also known as nylon, such as nylon 6 and nylon 6,6), polyester (e.g., polyethylene terephthlate), polytetrafluoroethylene (e.g., expanded polytetrafluoroethylene), polyether-ester such as polybutester (block copolymer of butylene terephthalate and polytetra methylene ether glycol), polyurethane, metal alloys, metal (e.g., stainless steel wire), polypropylene, polyethelene, silk, and cotton. Sutures made of non-degradable suture material are suitable for applications in which the suture is meant to remain permanently or is meant to be physically removed from the body.

"Suture diameter" refers to the diameter of the body of the suture. It is to be understood that a variety of suture lengths may be used with the sutures described herein and that while the term "diameter" is often associated with a circular periphery, it is to be understood herein to indicate a cross-sectional dimension associated with a periphery of any shape. Suture sizing is based upon diameter. United States Pharmacopoeia ("USP") designation of suture size runs from 0 to 7 in the larger range and 1-0 to 11-0 in the smaller range; in the smaller range, the higher the value preceding the hyphenated zero, the smaller the suture diameter. The actual diameter of a suture will depend on the suture material, so that, by way of example, a suture of size 5-0 and made of collagen will have a diameter of 0.15 mm, while sutures having the same USP size designation but made of a synthetic absorbable material or a non-absorbable material will each have a diameter of 0.1 mm. The selection of suture size for a particular purpose depends upon factors such as the nature of the tissue to be sutured and the importance of cosmetic concerns; while smaller sutures may be more easily manipulated through tight surgical sites and are associated with less scarring, the tensile strength of a suture manufactured from a given material tends to decrease with decreasing size. It is to be understood that the sutures and methods of manufacturing sutures disclosed herein are suited to a variety of diameters, including without limitation 7, 6, 5, 4, 3, 2, 1, 0, 1-0, 2-0, 3-0, 4-0, 5-0, 6-0, 7-0, 8-0, 9-0, 10-0 and 11-0.

"Suture deployment end" refers to an end of the suture to be deployed into tissue; one or both ends of the suture may be suture deployment ends. The suture deployment end may be attached to a deployment device such as a suture needle, or may be sufficiently sharp and rigid to penetrate tissue on its own.

"Armed suture" refers to a suture having a suture needle on at least one suture deployment end.

"Needle attachment" refers to the attachment of a needle to a suture requiring same for deployment into tissue, and can include methods such as crimping, swaging, using adhesives, and so forth. The suture thread is attached to the suture needle using methods such as crimping, swaging and adhesives. Attachment of sutures and surgical needles is described in U.S. Patent Nos. 3,981,307, 5,084,063, 5,102,418, 5,123,911, 5,500,991, 5,722,991, 6,012,216, and 6,163,948, and U.S. Patent Application Publication No. US 2004/0088003). The point of attachment of the suture to the needle is known as the swage.

"Suture needle" refers to needles used to deploy sutures into tissue, which come in many different shapes, forms and compositions. There are two main types of needles, traumatic needles and atraumatic needles. Traumatic needles have channels or drilled ends (that is, holes or eyes) and are supplied separate from the suture thread and are threaded on site. Atraumatic needles are eyeless and are attached to the suture at the factory by swaging or other methods whereby the suture material is inserted into a channel at the blunt end of the needle which is then deformed to a final shape to hold the suture and needle together. As such, atraumatic needles do not require extra time on site for threading and the suture end at the needle attachment site is generally smaller than the needle body. In the traumatic needle, the thread comes out of the needle's hole on both sides and often the suture rips the tissues to a certain extent as it passes through. Most modern sutures are swaged atraumatic needles. Atraumatic needles may be permanently swaged to the suture or may be designed to come off the suture with a sharp straight tug. These "pop-offs" are commonly used for interrupted sutures, where each suture is only passed once and then tied. For barbed sutures that are uninterrupted, these atraumatic needles are preferred.

Suture needles may also be classified according to the geometry of the tip or point of the needle. For example, needles may be (i) "tapered" whereby the needle body is round and tapers smoothly to a point; (ii) "cutting" whereby the needle body is triangular and has a sharpened cutting edge on the inside; (iii) "reverse cutting" whereby the cutting edge is on the outside; (iv) "trocar point" or "taper cut" whereby the needle body is round and tapered, but ends in a small triangular cutting point; (v) "blunt" points for sewing friable tissues; (vi) "side cutting" or "spatula points" whereby the needle is flat on top and bottom with a cutting edge along the front to one side (these are typically used for eye surgery).

Suture needles may also be of several shapes including, (i) straight, (ii) half curved or ski, (iii) 1/4 circle, (iv) 3/8 circle, (v) 1/2 circle, (vi) 5/8 circle, (v) and compound curve.

Suturing needles are described, for example, in US Patent Nos. 6,322,581 and 6,214,030 (Mani, Inc., Japan); and 5,464,422 (W.L. Gore, Newark, DE); and 5,941,899; 5,425,746; 5,306,288 and 5,156,615 (US Surgical Corp., Norwalk, CT); and 5,312,422 (Linvatec Corp., Largo, FL); and 7,063,716 (Tyco Healthcare, North Haven, CT). Other suturing needles are described, for example, in US Patent Nos. 6,129,741; 5,897,572; 5,676,675; and 5,693,072. The sutures described herein may be deployed with a variety of needle types (including without limitation curved, straight, long, short, micro, and so forth), needle cutting surfaces (including without limitation, cutting, tapered, and so forth), and needle attachment techniques (including without limitation, drilled end, crimped, and so forth). Moreover, the sutures described herein may themselves include sufficiently rigid and sharp ends so as to dispense with the requirement for deployment needles altogether.

"Needle diameter" refers to the diameter of a suture deployment needle at the widest point of that needle. While the term "diameter" is often associated with a circular periphery, it is to be understood herein to indicate a cross-sectional dimension associated with a periphery of any shape.

"Wound closure" refers to a surgical procedure for closing of a wound. An injury, especially one in which the skin or another external or internal surface is cut, torn, pierced, or otherwise broken is known as a wound. A wound commonly occurs when the integrity of any tissue is compromised (e.g., skin breaks or bums, muscle tears, or bone fractures). A wound may be caused by an act, such as a puncture, fall, or surgical procedure; by an infectious disease; or by an underlying medical condition. Surgical wound closure facilitates the biological event of healing by joining, or closely approximating, the edges of those wounds where the tissue has been torn, cut, or otherwise separated. Surgical wound closure directly apposes or approximates the tissue layers, which serves to minimize the volume new tissue formation required to bridge the gap between the two edges of the wound. Closure can serve both functional and aesthetic purposes. These purposes include elimination of dead space by approximating the subcutaneous tissues, minimization of scar formation by careful epidermal alignment, and avoidance of a depressed scar by precise eversion of skin edges.

'Tissue elevation procedure" refers to a surgical procedure for repositioning tissue from a lower elevation to a higher elevation (i.e. moving the tissue in a direction opposite to the direction of gravity). The retaining ligaments of the face support facial soft tissue in the normal anatomic position. However, with age, gravitational effects and loss of tissue volume effect downward migration of tissue, and fat descends into the plane between the superficial and deep facial fascia, thus causing facial tissue to sag. Face-lift procedures are designed to lift these sagging tissues, and are one example of a more general class of medical procedure known as a tissue elevation procedure. More generally, a tissue elevation procedure reverses the appearance change that results from effects of aging and gravity over time, and other temporal effects that cause tissue to sag, such as genetic effects. It should be noted that tissue can also be repositioned without elevation; in some procedures, tissues are repositioned laterally (away from the midline), medially (towards the midline) or inferiorly (lowered) in order to restore symmetry (i.e. repositioned such that the left and right sides of the body "match").

"Medical device" or "implant" refers to any object placed in the body for the purpose of restoring physiological function, reducing/alleviating symptoms associated with disease, and/or repairing and/or replacing damaged or diseased organs and tissues. While normally composed of biologically compatible synthetic materials (e.g., medical-grade stainless steel, titanium and other metals or polymers such as polyurethane, silicon, PLA, PLGA and other materials) that are exogenous, some medical devices and implants include materials derived from animals (e.g., "xenografts" such as whole animal organs; animal tissues such as heart valves; naturally occurring or chemically-modified molecules such as collagen, hyaluronic acid, proteins, carbohydrates and others), human donors (e.g., "allografts" such as whole organs; tissues such as bone grafts, skin grafts and others), or from the patients themselves (e.g., "autografts" such as saphenous vein grafts, skin grafts, tendon/ligament/muscle transplants). Medical devices that can be used in procedures in conjunction with the present invention include, but are not restricted to, orthopedic implants (artificial joints, ligaments and tendons; screws, plates, and other implantable hardware), dental implants, intravascular implants (arterial and venous vascular bypass grafts, hemodialysis access grafts; both autologous and synthetic), skin grafts (autologous, synthetic), tubes, drains, implantable tissue bulking agents, pumps, shunts, sealants, surgical meshes (e.g., hernia repair meshes, tissue scaffolds), fistula treatments, spinal implants (e.g., artificial intervertebral discs, spinal fusion devices, etc.) and the like.

### Materials for Shape-memory self-retaining sutures

As discussed above, the present invention provides compositions, configurations, for shape-memory self-retaining sutures in surgical procedures which greatly increase the ability of the self-retaining sutures to anchor into the surrounding tissue to provide superior holding strength and improve clinical performance.

Shape-memory materials are materials which have the ability to memorize one or more shapes different than the current temporary shape and transition between the shapes in response to a stimulus. A wide variety of materials are known to exhibit shape-memory effects including, for example, polymers, thermoplastics, metal alloys, hydrogels and ceramics. A shape-memory material transitions from one shape to another shape in response to a transition stimulus - typically a temperature change. However, a wide variety of different transition stimuli can be used to effect transition between shapes in shape-memory materials including, for example, temperature, electromagnetic radiation, electrical current, magnetic fields, pH, and solvation (including, but not limited to water solvation). The transition stimulus is typically dependent upon the shape-memory material.

Shape-memory metal alloys are known that transition between shapes in response to a temperature stimulus. Nickel-Titanium alloy known as NITINOL is a superelastic shape-memory alloy that is often used in medical applications. Nitinol is typically composed of approximately 55% Nickel by weight but making small changes in the composition can change the transition temperature of the alloy significantly. The temperature stimulus causes a transition in crystalline structure. When NITINOL is deformed below its transition temperature it will remain in the deformed shape until heated above its transition temperature, at which time it will return to its original shape. The shape-memory property of NITINOL is due to a temperature-dependent phase transformation from a low-symmetry to a high symmetry crystallographic structure. Those crystal structures are known as martensite (at lower temperatures) and austenite (at higher temperatures).

Shape-memory alloys may have different kinds of shape-memory effect including one-way and two-way shape-memory. In the one-way shape-memory effect a piece of the alloy transitions from a first shape at the low temperature to a permanent shape at a temperature above the transition temperature but does not substantially change shape upon returning below the transition temperature. In the two way shape-memory effect the alloy may regain some of the first shape upon returning below the transition temperature. Shape-memory metals include for example the alloys of: Ni-Ti, Ag-Cd, Au-Cd, Cu-Al-Ni, Cu-Sn, Cu-Zn, Cu-Zn-Si, Fe-Pt, Mn-Cu, and Fe-Mn-Si in suitable ratios.

Shape-memory polymers also transition between shapes in response to an external stimulus. Polymers with shape-memory have a current shape and one or more stored shapes. Dual-shape and triple-shape polymers have been described. A first stored shape is produced by conventional methods and the configuration of that shape is largely determined by covalent bonds between the polymer chains. The material is then deformed into a second (and possibly third) temporary form under conditions in which the second (and possibly third) form is fixed by mechanical connections made by particular "switching segment". The transformation into the second (and possibly third) temporary forms is a process called programming. The polymer maintains the second (and possibly third) form until another of the shapes is recalled by a predetermined external stimulus. Shape-memory polymers including dual-shape and triple-shape polymers are disclosed for example in U.S. Patents 6,160,084, 6,388,043, 6,720,402, 6,852,825, 7,037,984 and U.S. Patent Publications 2004/0014929, 2006/0116503, 2006/0140999, 2006/0257629, 2007/0088135 to Lendlein et al.

The shape-memory effect in shape-memory-polymers is due to a heterogeneous molecular network structure, which contains meltable "switching segments" among other harder segments. Applying a stimulus causes the crystallized switching segments to "melt" and the material recovers a form controlled ("memorized") by other elements of the crystalline structure of the polymer. A shape-memory polymer may have multiple different "switching segments" which melt in response to different stimuli thus permitting a plurality of shapes to be memorized. The transition stimulus for shape memory polymers is often temperature, but can also be an electric field, magnetic field, electromagnetic radiation (e.g. light), pH change, chemical change or solvation change (e.g. water). In some instances the transition stimulus is a means for raising the temperature of the polymer. Passing an electric current through a conductive polymer can be used to raise the temperature by resistive heating. Applying a fluctuating magnetic field to a polymer can be used to heat a polymer having ferromagnetic properties. Illuminating a polymer with electromagnetic radiation can be used to heat a polymer which absorbs (or is treated to absorb) the particular wavelength used. However, other transition stimuli such as pH, chemical change and solvation may be used to "melt" the switching segment without a change in temperature.

A shape memory material including a polymer or alloy may be incorporated in a self-retaining suture to provide shape memory attributes to the filament and/or retainers of the self-retaining suture. The particular shape-memory material selected depends upon the shape change desired and also choosing a transition stimulus compatible with the application. For example, where a shape-memory material is desired to undergo a shape-change after implantation in tissue, it is desirable that the transition stimulus be applied without damaging the tissue in which the material is implanted. In some embodiments, the material is selected such that the transition stimulus is a physiological property of the tissue in which the material will be implanted - such that the material undergoes a shape change automatically upon implantation in the tissue. In other embodiments the material is selected such that the transition stimulus is not a physiological property of the tissue in which the material will be implanted-such that the transition stimulus must be supplied to the material from an external device before the material undergoes a shape change.

### Shape-Memory Self-Retaining Suture Systems

The present invention provides compositions, for self retaining sutures and methods of using shape-memory self-self-retaining sutures and methods of using shape-memory self-retaining sutures in surgical procedures Shape-memory self-retaining sutures incorporate a shape-memory material in a way which affects the geometry of features of the shape-memory filament before, during or after deployment of the suture into tissue.

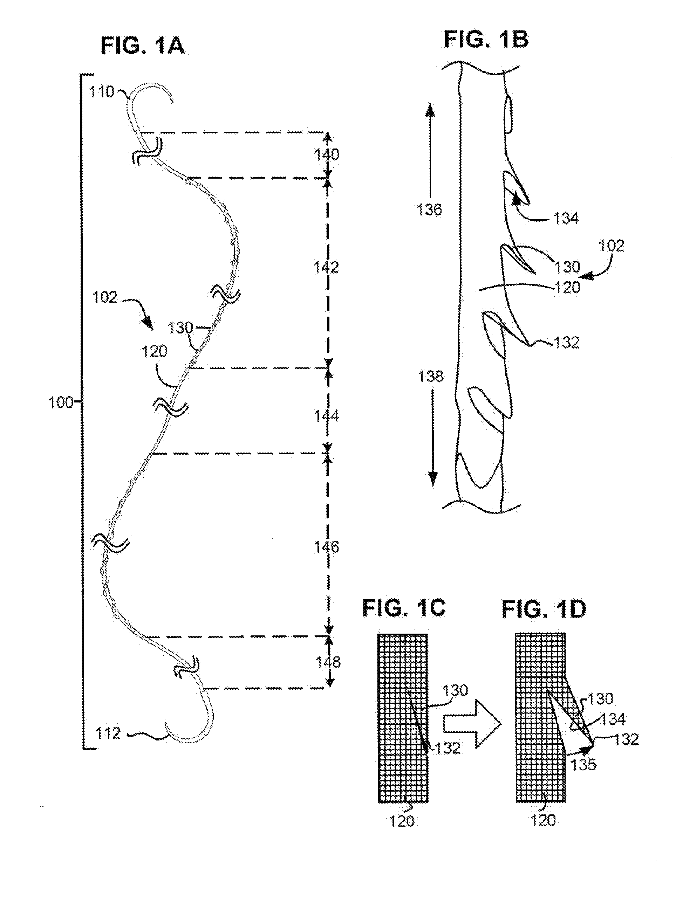

FIG. 1A illustrates a shape-memory self-retaining suture system 100. Shape-memory self-retaining suture system 100 is a bidirectional self-retaining suture and comprises needles 110, 112 attached at each end of shape-memory self-retaining suture thread 102. Shape-memory self-retaining suture thread 102 includes a plurality of retainers 130 distributed on the surface of a filament 120. In lead-in region 140 of shape-memory suture thread 102 there are no retainers 130. In region 142 of shape-memory suture thread 102 there are a plurality of retainers 130 arranged such that, when retainers 130 are elevated, the suture thread can be deployed in the direction of needle 110 but resists movement in the direction of needle 112. In transition region 144, there are no retainers 130. In region 146, there are a plurality of retainers 130 arranged such that, when retainers 130 are elevated, the suture thread can be deployed in the direction of needle 112 but resists movement in the direction of needle 110. In lead-in region 148 of self-retaining suture thread 102 there are no retainers 130. A break is shown in each of regions 140, 142, 144, 146 and 148 to indicate that the length of each region may be varied and selected depending upon the application for which the suture is intended to be used. Although a bidirectional shape-memory self-retaining suture system 100 is illustrated, the present invention includes self-retaining suture systems of a wide variety of retainer and needle configurations described above. Likewise the configuration of each of needles 110 and 112 can be any of the range of different surgical needles developed for use in different applications. Needles 110 and 112 may have the same configuration or different configurations.

FIG. 1B illustrates a magnified view of shape-memory self-retaining suture thread 102 in region 142. As shown in FIG. 1B, a plurality of retainers 130 is distributed on the surface of filament 120. The affixation of self-retaining sutures after deployment in tissue entails the penetration of retainer ends into the surrounding tissue resulting in tissue being caught between the retainer and the suture body. The inner surface of the retainers 130 contacts tissue that is caught between the retainers 130 and the filament 120. The inner surface of the retainers is referred to herein as the "tissue engagement surface" or "inner retainer surface." As illustrated in FIG. 1B, each retainer 130 has a tip 132 and tissue retainer surface 134.

In a shape-memory self-retaining suture system, the retainers 130 and/or filament 120 includes a shape-memory component. In some embodiments, the shape-memory component may be used to cause retainers 130 to elevate or lie flat against filament 120 in response to a stimulus. With the retainer 130 elevated, when self-retaining suture thread 102 is moved in the direction of arrow 138, tip 132 or retainer 130 engages tissue surrounding filament 120 and causes retainers 130 to fan out further from filament 120 and engage the tissue with tissue retainer surface 134 thereby preventing movement of the suture in that direction. However, prior to elevation of retainer 130 it may be possible to move shape-memory suture thread 102 in both of directions 136, 138 without interference by retainers 130. Thus, the use of shape-memory suture thread 102 allows for selectable control of the self-retaining features of the shape-memory self-retaining suture system 100.

FIG. 1C shows a cross-sectional view of shape-memory suture thread 102 in accordance with an embodiment of the invention. FIG. 1C shows one shape of shape-memory suture thread 102 in which retainer 130 lies flat against the body of filament 120. FIG. 1D shows a memorized or programmed shape of shape-memory suture thread 102 in which retainer 130 is elevated above the surface of filament 120 with tip 132 and tissue retainer surface 134 in position to engage tissue. Upon the application of a transition stimulus retainer 130 changes from the configuration of FIG. 1C to the configuration of FIG. 1D. Retainer tip 132 thus moves in the direction of arrow 135 away from filament 120 in response to the transition stimulus. The transition stimulus may be, for example, causing the shape-memory filament 120 and/or retainers 130 to reach a transition temperature. The transition temperature may be selected to be a physiologic tissue temperature such that the retainer will automatically elevate after positioning in tissue. Alternatively, the transition temperature may be selected to be a temperature above physiologic tissue temperature such that an external source of heat must be provided in order to elevate the retainers 130. The particular transition temperature may be selected by modifying the composition of the shape-memory material in filament 120 and retainers 130.

### Shape-Memory Self-Retaining Suture Formation, Prograimming and Actuation

Shape-memory suture threads as described herein may be produced by any suitable method, including without limitation, one or a combination of injection molding, stamping, cutting, laser, extrusion, and so forth. With respect to cutting, polymeric thread or filaments may be manufactured or purchased for the suture body, and the retainers can be subsequently cut onto the suture body. Such retainers may be hand-cut, laser-cut, or mechanically machine-cut using blades, cutting wheels, grinding wheels, and so forth. During cutting either the cutting device or the suture thread may be moved relative to the other, or both may be moved, to control the size, shape and depth of cut. Particular methods for cutting barbs on a filament are described in U.S. Patent Application Serial No. 09/943,733 titled "Method Of Forming Barbs On A Suture And Apparatus For Performing Same" to Genova et al., and U.S. Patent Application Serial No. 10/065,280 titled "Barbed Sutures" to Leung et al.

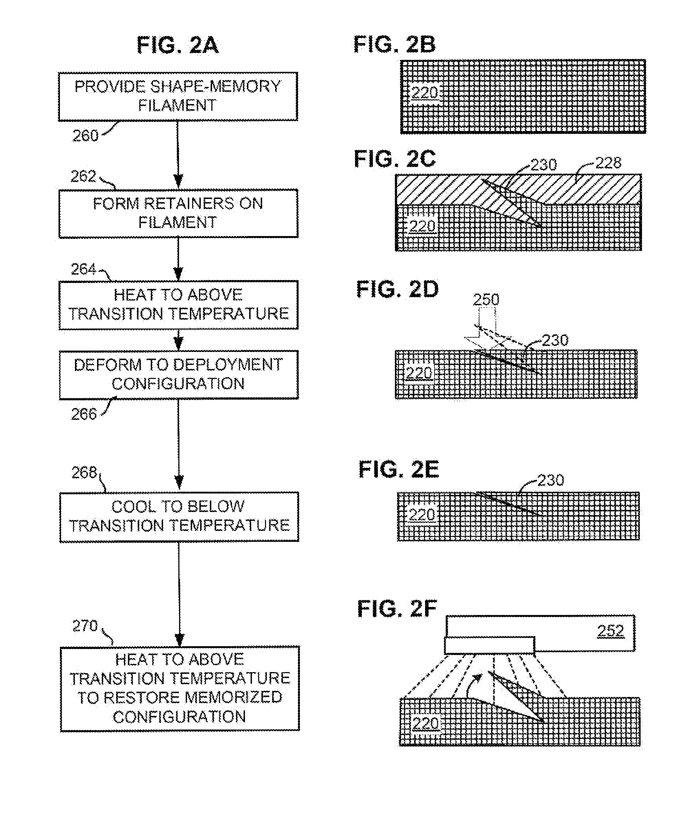

FIG. 2A is a flow chart demonstrating an exemplary process for making a shape-memory suture thread 102 having a retainer 230 on a filament 220. FIGS. 2B through 2F illustrate steps of the process of FIG. 2A. In step 260 of FIG. 2A, a filament 220 including a shape-memory material is provided. In the present example, filament 220 is made of a shape-memory polymer. The shape-memory polymer is selected and/or designed to have a suitable transition temperature, which may, for example be body temperature, but in this case is a temperature a few degrees above body temperature. FIG. 2B shows a sectional view of the filament 220 before retainer formation.

In step 262 of FIG. 2A, a retainer 230 is formed in the filament 220. Retainer 230 may be formed by using any of a wide range of technologies. FIG. 2C shows a sectional view of the filament 220 and retainer 230. Note that in this embodiment, retainer 230 has been formed by removing material 228 from filament 220. Material 228 can be removed using any of a wide range of technologies including without limitation: hand-cutting, laser-cutting, stamping or machine-cutting using blades, cutting wheels, grinding wheels, and so forth. As shown in FIG. 2C, retainer 230 as formed, has a configuration which is elevated above the body of filament 220. The elevated configuration of retainer 230 is, in this case, the configuration controlled by the covalent cross-linking of the shape-memory polymer of which retainer 230 is made.

In an alternative embodiment, a retainer 230 may be formed in filament 220 by making a cut into the surface of filament 220. When a retainer 230 is made in this manner, the retainer needs to be elevated above the surface of filament in order to engage tissue. The retainer may be annealed while held in the elevated position at a temperature sufficient to alter the permanent configuration of the filament. The retainers may be elevated using a range of different technologies, including mechanically lifting each retainer and wrapping the filament around a mandrel. Technology for elevating retainers is disclosed in United States Provisional Patent Application 61/030,423 entitled "Method And Apparatus For Elevating Retainers On Self-Retaining Sutures" to Goraltchouk et al.

In steps 264, 266 and 268, of FIG. 2A retainer 230 is deformed into a temporary configuration. As shown in step 264 of FIG. 2A, in order to deform retainer 230 into a temporary deployment configuration, retainer 230 is first raised to a temperature above the transition temperature. Next, in step 266, retainer 230 is deformed into the temporary configuration by application of a deforming force 250 while maintaining the temperature above the transition temperature. FIG. 2D shows the deformation of the retainer 230 from its original position (shown by the dotted line) in which the retainer is elevated above the surface of filament 220 to a temporary configuration in which the retainer 230 is flush with the surface of filament 220.

In step 268 of FIG. 2A the filament is cooled to below the transition temperature while maintaining retainer 230 in the temporary configuration by application of force 250. In the cooling step 268 the meltable switching segments of the shape-memory polymer freeze creating mechanical linkages that fix the shape-memory polymer in the temporary shape. Thus, at the end of step 268, the force 250 deforming retainer 230 is released and the retainer 230 remains in the temporary configuration shown in FIG. 2E in which retainer 230 lies flat against filament 220.

Retainer 230 will remain flush against filament 220 in the temporary configuration shown in FIG. 2E until exposure to the transition stimulus. With retainer 230 flush against filament 220, filament 220 may be deployed in either direction without retainer 230 engaging the tissue. In step 270 a transition stimulus is provided. The transition stimulus causes melting of the meltable switching segments of the shape-memory polymer and unfreezes the temporary configuration. When the temporary configuration is melted, retainer 230 returns to the permanent configuration controlled by the covalent cross-linking of the shape-memory polymer of which retainer 230 is made. Thus, retainer 230 returns to the elevated configuration as shown in FIG. 2F.

### Applications of Self-Retaining Sutures with Shape-Memory Properties

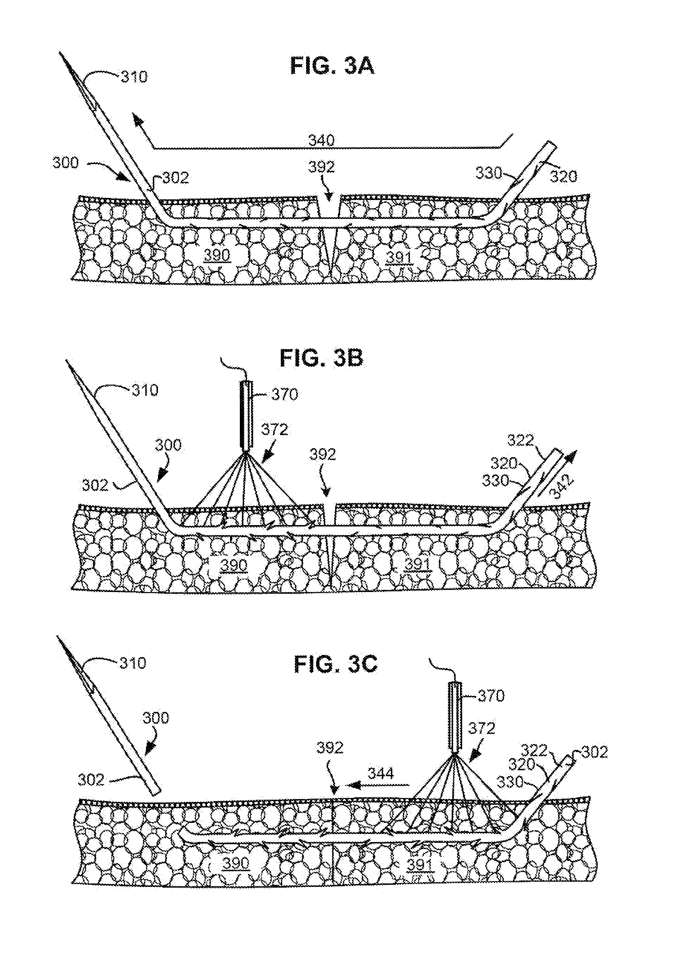

FIGS. 3A, 3B and 3C illustrate a method of using a shape-memory self-retaining suture having shape-memory retainers in a procedure to close a wound. As shown in FIG. 3A, self-retaining suture system 300 comprises a needle 310 attached to a self-retaining suture thread 302 comprising a filament 320 and a plurality of retainers 330. In order to close wound 392 dividing tissue 390 from tissue 391 needle 310 is drawn through tissue 391, across wound 392, and then through tissue 390 all in the direction of arrow 340. During this deployment, the plurality of retainers 330 are in a temporary (or deployment) configuration in which they are lying flat against the body of filament 320. Retainers 330 thus do not obstruct the movement of filament 320 in either direction. Thus filament 320 may be deployed in either direction and removed and/or repositioned as necessary without damaging the tissue 390 and 391 through which it passes.

As shown in FIG. 3B, after the shape-memory self-retaining suture system 300 has been deployed through the tissue 390, 391 on either side of wound 392 a transition stimulus is selectively applied to the portion of the self-retaining suture thread 302 lying in tissue 390. As shown in FIG. 3B the transition stimulus is provided by electromagnetic radiation source 370 (IR, visible light UV and the like) which provides electromagnetic radiation 372 in a directable manner. The wavelength of electromagnetic radiation 372 is selected such that it is preferentially absorbed by filament 320 rather than tissue 390 and thus may pass through tissue 390 without injuring tissue 390. The region of filament 320 exposed to the electromagnetic radiation 372 is elevated to a temperature above the transition temperature. As a result, the retainers 330 in the region of filament 320 exposed to the electromagnetic radiation 372 return to the permanent (or deployed) configuration in which they are elevated above the surface of filament 320. Retainers 330 in the region of filament 320 exposed to the electromagnetic radiation 372 thus engage tissue 390. Free end 322 of self-retaining suture thread 302 can then be drawn in the direction of arrow 342. Because retainers 330 have engaged tissue 390, applying tension to free end 322 of self-retaining suture thread 302 pulls tissue 390 closer to tissue 391, closing wound 392.

As shown in FIG. 3C, needle 310 and the end of self-retaining suture thread 302 may be removed. Electromagnetic source 370 is then used to provide electromagnetic radiation to the filament 320 within tissue 391. When electromagnetic radiation 372 is absorbed by filament 320 in tissue 391 the filament 320 in the region exposed to the electromagnetic radiation 372 is elevated to a temperature above the transition temperature. As a result, the retainers 330 in the region of filament 320 exposed to the electromagnetic radiation 372 return to the permanent configuration in which the retainers 330 are elevated above the surface of filament 320. Retainers 330 in the region of filament 320 exposed to the electromagnetic radiation 372 thus engage tissue 391. Tissue 391 may be manually advanced or grouped over filament 320 in the direction of arrow 344 closing wound 392 completely. Free end 322 of filament 320 may then be removed. The retainers 330 engage tissue 390 and tissue 391 holding the tissue together and closing wound 392. No suture knot is required.

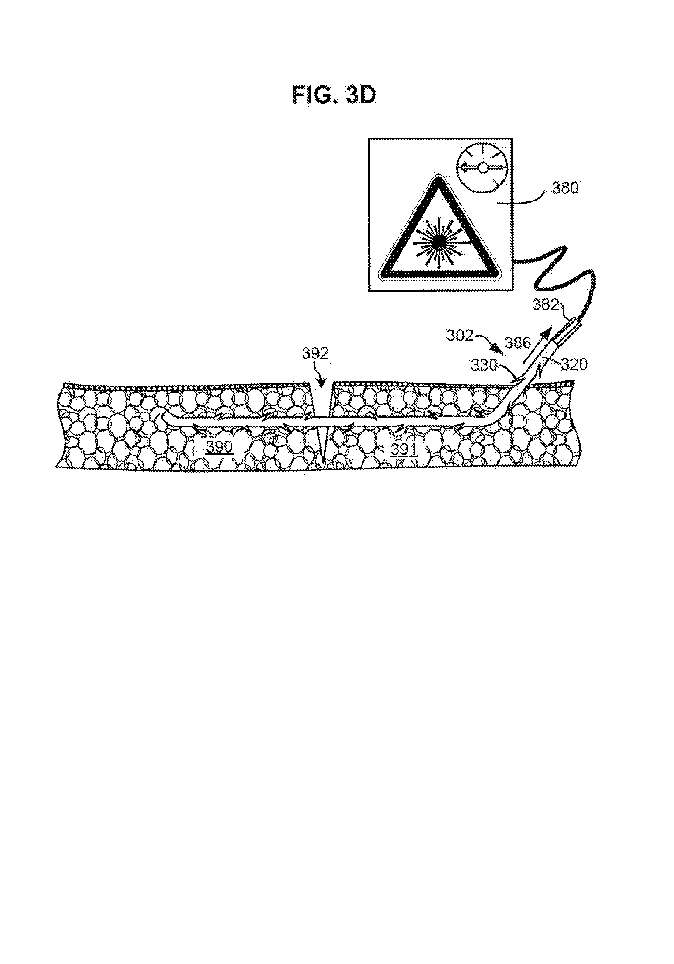

In another alternative embodiment, also illustrated in FIG. 3D, a source 380 of electromagnetic radiation (such as UV, IR, visible light and the like) is connected to one end of the self-retaining suture thread 302 and travels through the filament 320. As shown in FIG. 3D the source of electromagnetic radiation may be, for example, a laser light source 380 coupled to one end of the self-retaining suture thread 302 with a fiber optic coupling 382. Filament 320 of self-retaining suture thread 302 acts as an optical fiber to transmit the laser light from laser light source 380 throughout filament 320. The light may travel through the material of filament 320 and retainers 330 depending upon the optical characteristics of the material and the wavelength of the light. The shape-memory material of the filament is selected such that sufficient light of the frequency required to melt the switching segments is transmitted along the length of self-retaining suture thread 302. This embodiment has the advantage that the light (or other electromagnetic radiation) radiation is delivered in a more targeted fashion to the retainers 330 and the transition stimulus need not pass through the tissue to reach all regions of the self-retaining suture thread 302.

As shown, in FIG. 3D, the light from laser light source 380 passes though the entire length of filament 320 and thus the transition stimulus is applied to all of the retainers 330 at the same time. Where all the retainers will be elevated at the same time, it is desirable in some embodiments to approximate the wound using clamps, clips, forceps or the like prior to elevation of the retainers. However, in the embodiment shown in FIG. 3D, wound 392 may be approximated after elevation of retainers 330 because the self-retaining suture thread 302 within tissue 391 may be pulled through the tissue in the direction of arrow 386 after elevation of retainers 330.

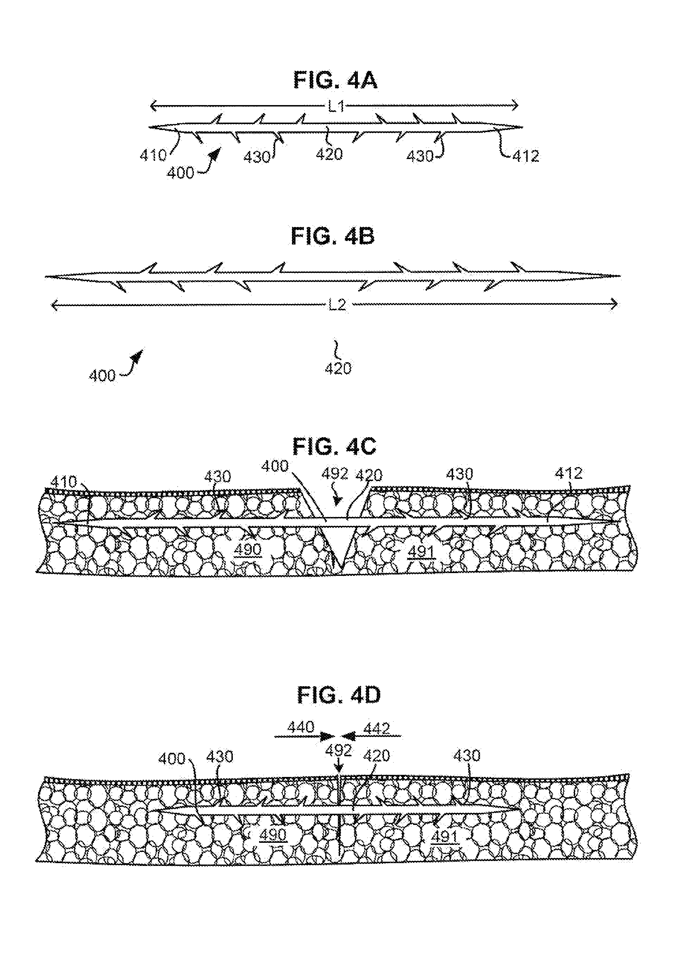

The shape-memory properties of a shape-memory suture thread can also be utilized to affect the size and/or shape of the filament instead of the retainers of the self-retaining suture. Shape-memory can be used to store a temporary filament shape instead of or in addition to using shape-memory to affect retainer elevation. Thus, in a simple example, the shape-memory effect may be used to reduce the length of a suture after deployment in order to approximate a wound. FIGS. 4A through 4H illustrate the use of the shape-memory effect to change the length of a shape-memory self-retaining suture after deployment into tissue.

FIG. 4A shows a bidirectional shape-memory self-retaining suture system 400. The system comprises a shape-memory filament 420, a plurality of retainers 430 and integrated needles 410, 412. Shape-memory filament 420 is made of a shape-memory polymer. The permanent shape of shape-memory filament 420 is as shown in FIG. 4A. The length of shape-memory filament 420 in the permanent shape is L1.

As shown in FIG. 4B, shape-memory filament 420 may be deformed by stretching the filament into a temporary shape having length L2. The temporary shape may be fixed as described above with respect to FIG. 2. The shape-memory self-retaining suture system 400 is stretched to length L2 at a temperature above the transition temperature for the shape-memory polymer. The shape-memory filament is then cooled to a temperature below the transition temperature while maintaining the filament in the stretched condition. After shape-memory filament 420 has been cooled below the transition temperature, the deforming forces may be removed and the filament will retain the temporary shape until raised above the transition temperature. The ratio L2 to L1 may be selected based upon the application intended for the shape-memory self-retaining suture system 400 but in general L1 may be from 20% to 99% of L2. More preferably, L1 is between 80% and 95% of L2.

FIG. 4C shows deployment of the shape-memory self-retaining suture system 400 across a wound 492 between tissue 490 and tissue 491. The shape-memory self-retaining suture system 400 is deployed in the temporary configuration having length L2. Needle 410 and one section of self-retaining suture system 400 is introduced into tissue 490 on the left side of wound 490. Retainers 430 in the section of suture introduced into tissue 490 are arranged such that the suture may pass through tissue in the direction of needle 410 but the retainers resist movement of the filament in the opposite direction. Needle 412 and the other section of self-retaining suture system 400 are introduced into tissue 491 on the right side of wound 490. Retainers 430 in the section of suture introduced into tissue 491 are arranged such that the suture may pass through tissue in the direction of needle 412 but the retainers resist movement of the filament in the opposite direction.

FIG. 4D shows transition of shape-memory self-retaining suture system 400 back to the stored permanent shape upon application of the transition stimulus. In this case, the transition stimulus may be heating the filament to a temperature at or above a transition temperature. When the filament 420 reaches the transition temperature, the filament begins to contract in the direction shown by arrows 440, 442. Retainers 430 fan out from filament 420 and catch tissue 490, 491 between the retainers and the filament 420. Thus, as filament 420 contracts, tissue 490 is drawn towards tissue 491 closing wound 492 as shown. A transition temperature between room temperature and body temperature may be of particular interest as the filament may be stored and deployed in one configuration below the transition temperature and then rises above the transition temperature upon deployment in the body causing it to recover another configuration. If the transition temperature is less than or equal to the temperature of tissue 490, 491, filament 420 will begin to contract as soon as it equilibrates in temperature with the surrounding tissue.

In alternative embodiments, an external stimulus may be required to cause elevation of the retainers. Such an external stimulus may be, for example, the application of heat to cause a temperature rise in the suture in excess of natural body temperature. The temperature rise can be caused by heating the suture outside the body prior to deployment. Alternatively, magnetic particles may be embedded in the material of the suture and caused to heat the suture material by magnetic induction caused by application of a magnetic filed through the tissue of the subject after deployment of the suture. Additionally, shape-memory polymers which contract upon application of UV light, pH or other stimuli which may be applied to the suture after deployment in the tissues may be used. In such cases, the transition stimulus may be controlled in order to cause all or part of filament 420 to contract thus a measure of control of the wound approximation and/or the force applied to tissues 490, 491 may be achieved.

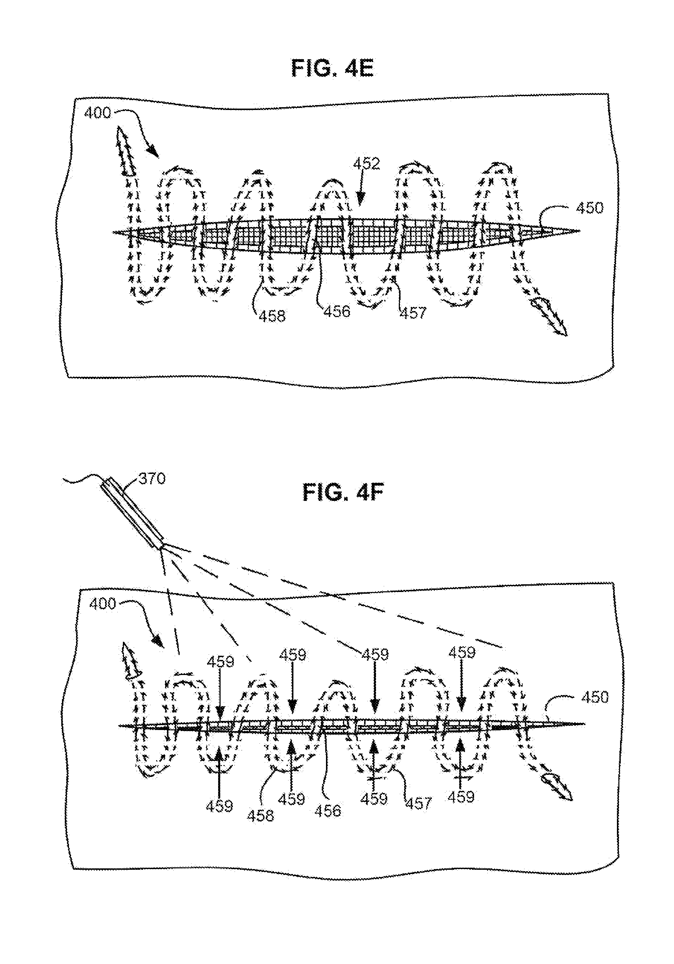

FIGS. 4E and 4F illustrate an alternative deployment of bidirectional shape-memory self-retaining suture 400 to close a wound 450. Note that wound 450 gapes in the center section 452. The wound 450 is closed from the center section 452. Note that the transition region 456 of the suture is located in the center of the wound 450 and each arm 457, 458 of suture 400 passes through the tissue along a sinuous path towards a different end of the wound. Because a shape-memory self-retaining suture 400 is used, the surgeon can avoid applying sufficient tension to completely close the wound during deployment of the suture. This is useful in a gaping wound which may require a large amount of tension to close. If a large amount of tension is applied when only one bite through the tissue has been made on each side of the wound, the tension may tear the suture from the tissue. Using the shape-memory self-retaining suture, the surgeon applies some tension to the suture during deployment but may leave the wound slightly open until the suture has been fully deployed as shown in FIG. 4E. As shown in FIG. 4E, suture 400 crosses wound 450 eleven times with suture 400 making five bites through tissue on each side of the wound.

As shown in FIG. 4F, after suture 400 has been deployed in wound 450, a transition stimulus may be supplied to generate further tension in suture 400 thereby closing the wound. A transition stimulus may be applied, for example by electromagnetic radiation source 370. In response to the transition stimulus, the suture 400 contracts pulling the wound 450 closed as shown by arrows 459. The additional tension caused by the shape-memory effect does not tear the tissue because the tension is now distributed over the entire length of the wound 450 rather than being directed only to the tissue at the center 452 of wound 450. Note that the transition stimulus may be applied to the entire length of suture 400 or may be applied selectively to different portions of suture 400 as needed to close wound 450. There could be stepwise activation, first a primary tightening to bring the tissues together and then a secondary contraction step allowing the tissue to relax and stretch in response to the tension rather than tear. The suture may be relaxed when deployed followed by a multi-step contraction. The entire length of the self-retaining suture works to bring the two edges of the wound together (unlike in the case of regular suturing, where each throw is used separately to approximate the edges of the wound). Alternatively, the transition stimulus can be applied by the heat of the tissue in which case, the surgeon, can apply a smaller level of tension with the initial few throws and while continuing to the next throws the segment which is already inserted will begin to contract and exert more force to bring the two edges of the wound closer together. This facilitates and expedites segmental closing of the wound.

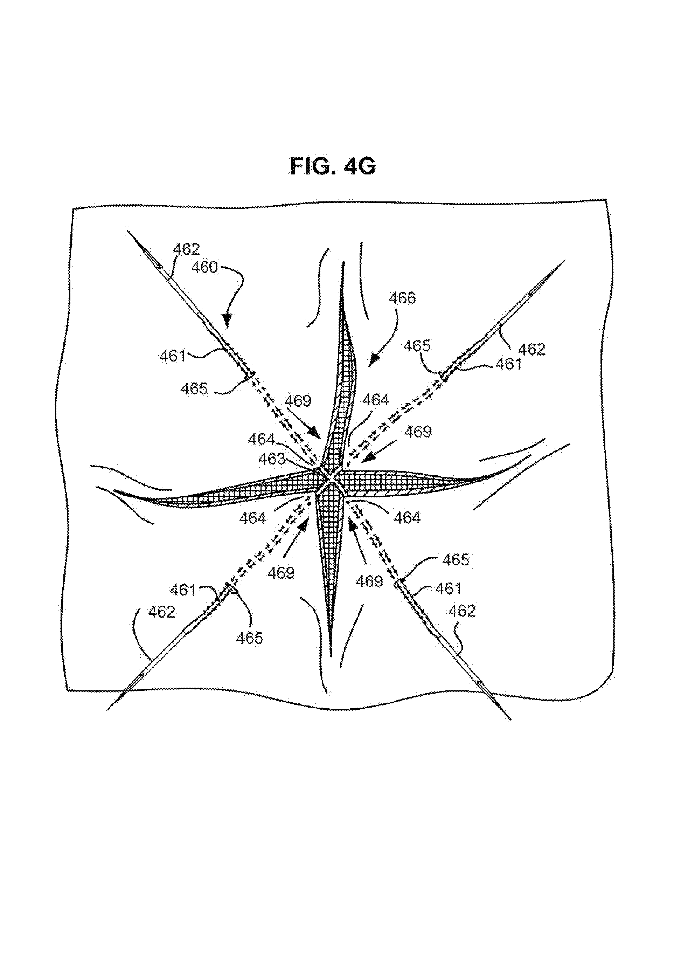

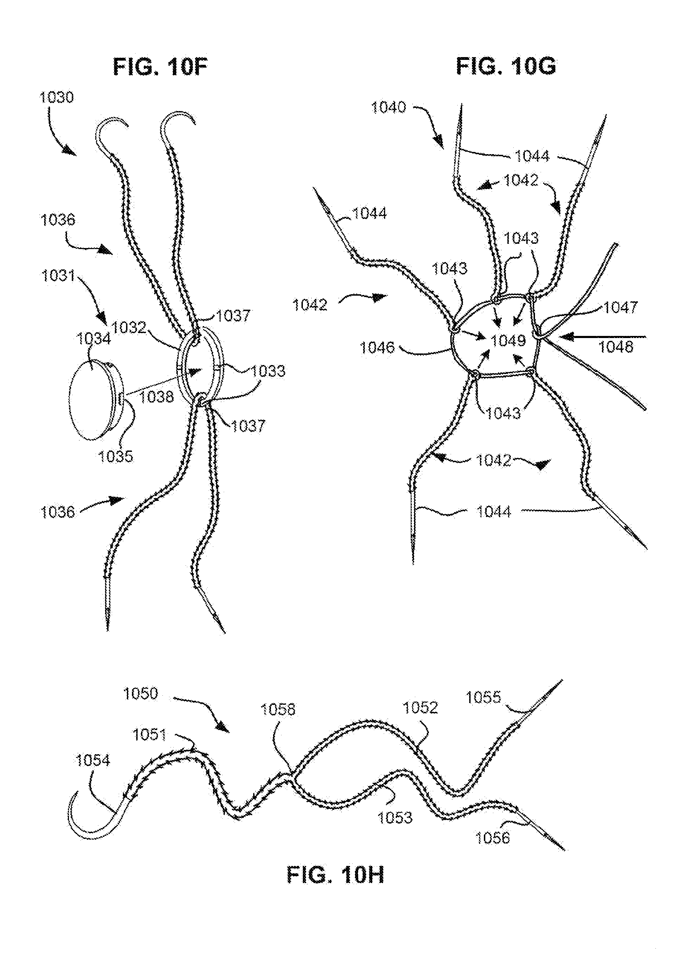

Self-retaining suture systems may comprise more than two arms. A self-retaining suture system may have one, two or more arms including up to about ten arms or more depending upon the application. For example, as shown in FIG. 4G a self-retaining suture system 460 comprises four arms 461 each having a needle 462 at one end and joined at a connection 463 at the other end. Connection 463 may be a separate connector component or may be a point where the four arms 461 are fused, joined or welded together. Where the connection is a separate connector component it may be made of the same materials described herein for the manufacture of sutures and may be made of an absorbable material. Alternatively two or more portions of suture may be knotted together at a connection point. Some alternative embodiments of self-retaining suture systems having more than two arms are described in FIGS. 10F-10H and accompanying text. The four arms 461 and connector 463 may also be formed in one piece. Self-retaining suture having more than two arms are useful in applications where it is desirable to have a plurality of suture lines radiating from a common point. Such self-retaining suture systems are useful for example in closes, puncture wounds, stellate wounds and other non-linear wounds. Such wounds can be produced by blunt trauma, gunshots, explosions and the like and are quite difficult to close with regular suturing techniques.

Self-retaining suture systems having more than two arms, such as shown in FIG. 4G are particularly suited for closing stellate wounds such as wound 466. Stellate wounds are nonlinear wounds where several tears through tissue meet at a common point and are difficult to close with regular suturing techniques. However, as shown in FIG. 4G, such a wound can be readily closed using a multi-arm self-retaining suture system having an arm for each tissue apex 464. Each needle 462 is inserted at the apex 464 of a tissue flap and drawn through the tissue to an exit point 465 located a distance away from the wound. Slight tension can be applied to the suture and the tissue manually urged over the suture in the direction shown by arrows 469 to begin closing the wound.

Self-retaining suture systems comprising more than two arms may be made using the shape-memory materials described herein and/or non-shape-memory materials. For example the self-retaining suture system 460 of FIG. 4G may be made of a shape-memory polymer and configured to shorten in length when a transition stimulus is applied to the suture. Thus, after the self-retaining suture system 460 has been deployed as shown in FIG. 4G, the transition stimulus may be applied causing the arms 461 to contract in the direction of arrows 469 drawing in each of tissue apexes 464 towards the connector 463 and closing the wound 460. After closing the central wound, the remaining linear wounds may be closed further if necessary using standard techniques. Shape-memory self-retaining suture systems having more than two arms, may also be useful for closing other complicated wounds where there is a need to draw the tissue towards a central point. For example where a portion of tissue is missing, it may also be desirable to draw the edges of the wound towards a central point using such a suture.

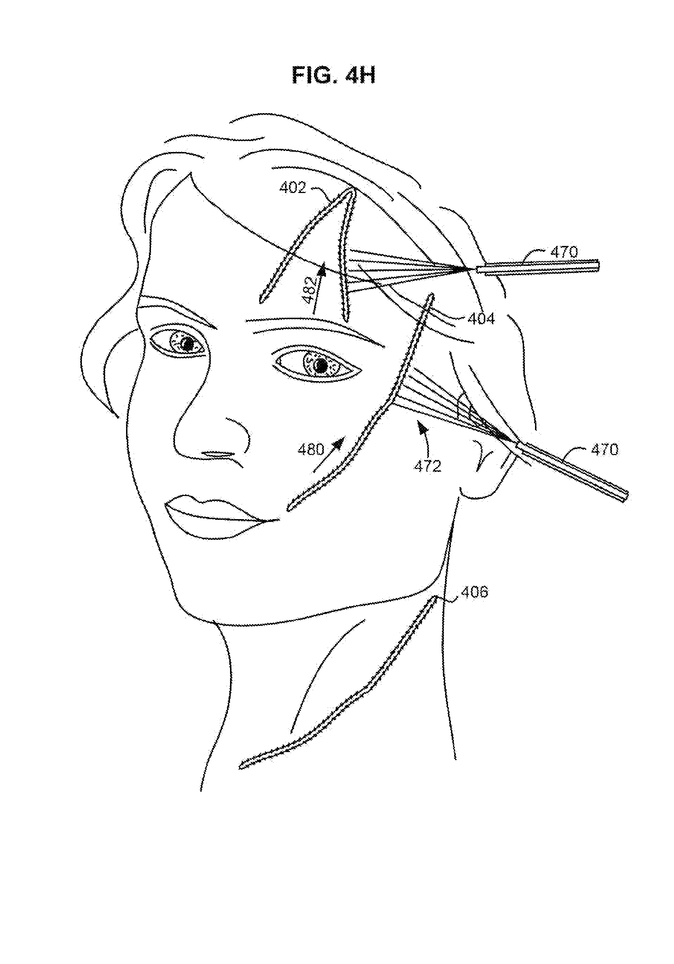

Shape-memory can be used to store a temporary filament shape instead of or in addition to using shape-memory to affect retainer elevation. Thus, in another simple example, the shape-memory effect may be used to elevate tissue after deployment. FIG. 4H shows the use of shape-memory properties of a shape-memory suture thread to affect the size and/or shape of the filament and thereby cause selective tissue elevation in response to selective application of a transition stimulus. FIG. 4H shows three bidirectional shape-memory self-retaining suture 402, 404, 406 deployed in the tissue of the face of a patient. Each of the sutures is anchored at an upper location either in soft tissue or by anchoring to a stable anatomical feature such as the fascia. As shown in FIG. 4H, after deployment of the shape-memory self-retaining sutures 402, 404, 406 into the face of the patient, a transition stimulus may be selectively applied to the suture through the skin. As shown in FIG. 4H, the transition stimulus is provided by electromagnetic radiation source 470 (IR, visible light UV and the like) which provides electromagnetic radiation 472 in a selectable and directable manner. The wavelength of electromagnetic radiation 472 is selected such that it is preferentially absorbed by shape-memory self-retaining sutures 402, 404, 406 rather than the facial tissue and thus may pass through the facial tissue without injuring the facial tissue. When the transition stimulus is applied to a localized region of the shape-memory self-retaining suture in this manner that region of the shape-memory self-retaining suture contracts to a smaller size. Because the upper region of the shape-memory self-retaining suture is firmly anchored, the lower portion of the shape-memory self-retaining suture is caused to move upwards. This causes elevation of the tissue in which the shape-memory self-retaining suture is embedded as shown by arrow 480, 482. The amount and location of the elevation may be controlled by selective application of the transition stimulus to different regions of the shape-memory self-retaining sutures 402, 404, 406.

A shape-memory self-retaining suture may be used for tissue elevation procedures in all parts of the body where tissue elevation procedures are performed. For example shape-memory self-retaining suture may be used in procedures, including, but not limited to face-lifts, brow-lifts, breast-lifts, neck-lifts, thigh lifts, and buttock lifts. As described with respect to FIG. 4H, the amount and location of the elevation may be controlled by selective application of the transition stimulus to different regions of the shape-memory self-retaining sutures used in the lift. Particular methods and patterns for deploying sutures for tissue elevation procedures are illustrated in U.S. Patent 7,056,331 entitled "Suture Method" to Kaplan et al. and U.S. Patent Application 2008/0082129 entitled "Minimally-Invasive Mastoplasty Procedure And Apparatus" to Jones et al.

The use of shape-memory self-retaining sutures in tissue elevation applications has many advantages. The elevation may be carried out some-time subsequent to the deployment step. For example, the suture may be deployed and then the patient released for a recovery period. Some time later, the patient returns to the physicians' office. The physician can apply the transition stimulus to the suture to elevate the tissue while the patient is watching in a mirror. Thus the patient can guide the physician to achieve the aesthetic effect desired by the patient. Moreover, if the transition stimulus is not applied to all regions of the shape-memory self-retaining suture, if further elevation is required at a later date, the patient may return and have the transition stimulus applied to further regions of the shape-memory self-retaining suture to cause more elevation. The time period between the deployment and elevation steps also allows the shape-memory self-retaining suture to become better anchored in the tissue prior to elevation allowing for a better result. Furthermore, swelling caused by suture deployment can dissipate prior to elevation such that the true effects of the elevation can be directly observed without masking by the effects of inflammation and swelling.

### Triple-Shape Shape-memory self-retaining suture

Triple-shape shape-memory polymers allow an article to be made which has two temporary shapes in addition to its permanent shape. Examples of such materials are described in Bellin et al., "Polymeric triple-shape materials" PNAS 103:48 pp. 18043-47 (2006). Triple-shape-memory polymers have two types of meltable switching segment. Each switching segment responds to a different transition stimulus. The article is formed from the melted polymer as previously described. The polymer is then raised to a temperature above the transition temperature for both of the switching segments. The article is then deformed to a first temporary shape and held in the first temporary shape while the temperature is reduced below the transition temperature of the first switching segment. The article is then deformed to a second temporary shape while the temperature is between the transition temperature of the first switching segments and the transition temperature of the second switching segments. The article is held in the second temporary shape while the temperature is reduced below the transition temperature of the second switching segment. After the article is cooled below the transition temperature of the second switching segment, the deforming force may be removed and the article will retain the second temporary shape. Subsequently a first external transition stimulus can raise the temperature of the article to a temperature above the transition temperature of the second switching segment. At this temperature, the second switching segment melts and the second temporary shape is lost and the first temporary shape is recovered. The article will retain this second temporary shape until a second external transition stimulus raise the temperature of the article to a temperature above the transition temperature of the first switching segment. At this temperature, the first switching segment melts and the first temporary shape is lost and the permanent shape is recovered.

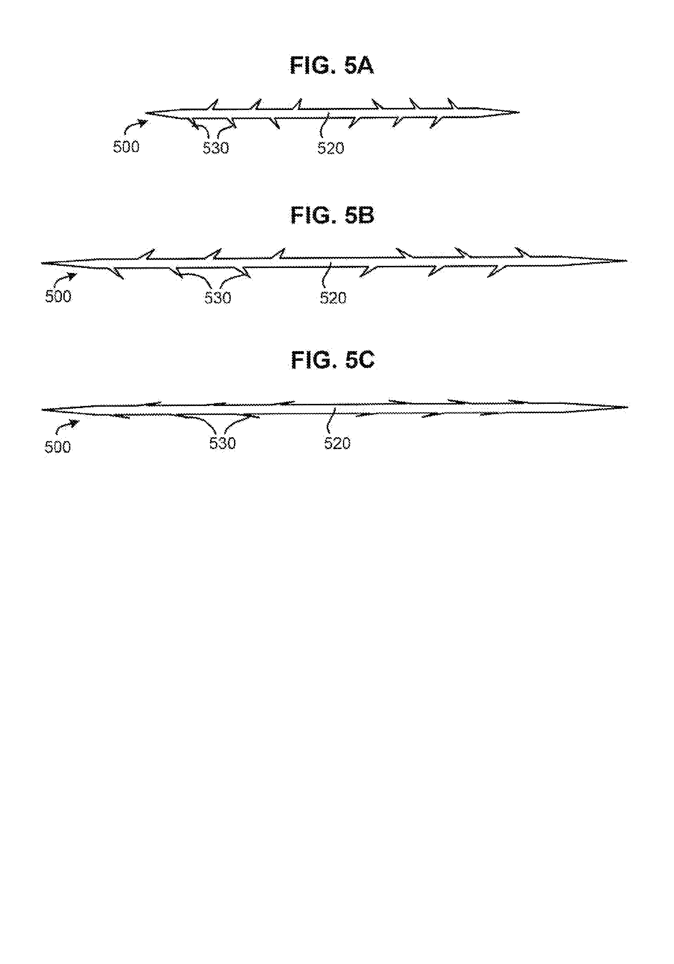

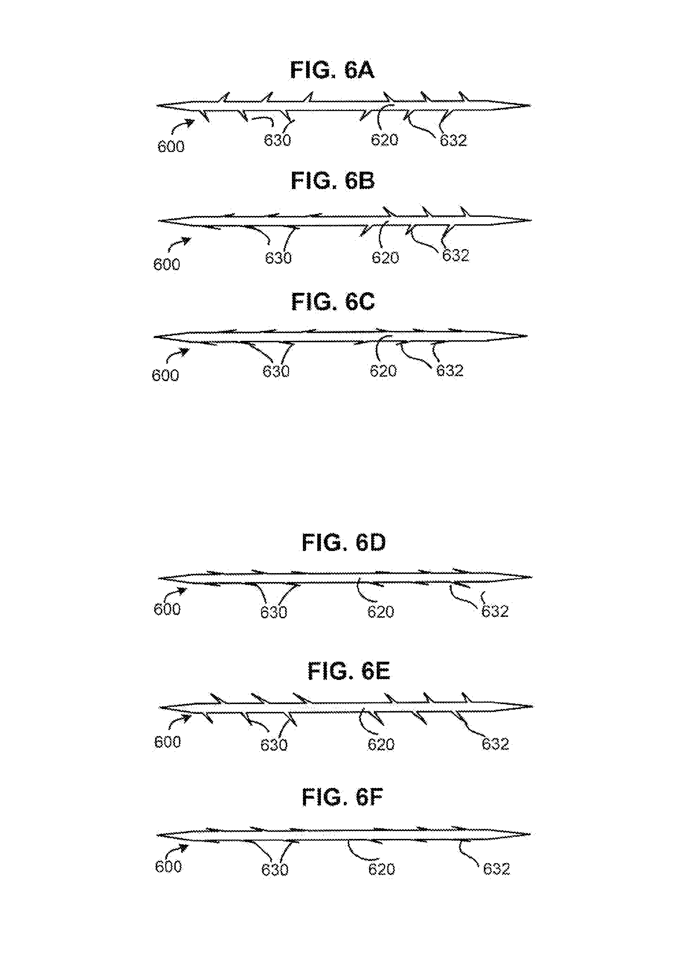

Examples of the use of triple-shape shape-memory polymers in self-retaining sutures are illustrated in FIGS. 5A-5C and 6A-6C. FIG. 5A shows a shape-memory self-retaining suture 500 in its permanent shape. Shape-memory self-retaining suture 500 is made from a filament of triple-shape shape-memory polymer. For purposes of this example, the triple-shape shape-memory polymer comprises two meltable switching segments. The first has a transition temperature of 42 °C the second has a transition temperature of 32 °C. The self-retaining suture 500 is raised to a temperature above 42 °C but below the melting point of the self-retaining suture 500. Self-retaining suture is then stretched as shown in FIG. 5B. Self-retaining suture 500 is held in the stretched shape shown in FIG. 5B while the temperature is reduced to 37 °C. At 37 °C the first switching segment has frozen. When the tension is removed, the self-retaining suture remains in the first temporary shape shown in FIG. 5B. While still at 37 °C, self-retaining suture 500 is deformed such that retainers 530 lay flat against filament 520. With the retainers held in this second temporary shape, the temperature of the suture is reduced to room temperature (20 °C). At this temperature, the second switching segment is frozen and when the forces deforming the retainers 530 are removed, the retainers stay flat against filament 520 as shown in FIG. 5C.

Self-retaining suture 500 is be deployed into tissues in the configuration shown in FIG. 5C. Upon deployment into tissue, the suture will rise to the ambient temperature of the tissues - approximately 37 °C. This temperature is above the second transition temperature and thus the suture will lose the second temporary shape shown in FIG. 5C and return to the shape shown in FIG. 5B. This transition causes the retainers 530 to fan out from filament 520 within a few seconds to a minute of suture 500 being deployed into tissue. Thus the retainers of self-retaining suture 500 automatically elevate upon deployment of the suture into tissue. Subsequently, an external transition stimulus can be applied to raise the temperature of shape-memory self-retaining suture 500 to 42 °C using a process which selectively heats filament 520 rather than the tissue. Upon reaching 42 °C, self-retaining suture 500 loses the first temporary shape of FIG. 5B and returns to the permanent shape of FIG. 5A. The suture 500 will thus shrink. Because retainers 530 are already deployed and engaged with tissue, the shrinking of self-retaining suture 500 will cause approximation of the tissue in which self-retaining suture 500 is deployed.

Many different programmed configurations of self-retaining suture made of triple-shape shape-memory polymers are possible. FIGS. 6A-6C illustrate a self-retaining suture comprises two sets of retainers 630 and 632. FIG. 6A shows the permanent shape in which self-retaining suture 600 is formed. In the first temporary shape, shown in FIG. 6B, the first set of retainers is caused to lie flat against filament 620. In the second temporary shape, shown in FIG. 6C, both sets of retainers 630, 632 are positioned flat against filament 620. The self-retaining suture can then be deployed in either direction in the second temporary configuration. The second set of retainers 632 may be elevated by providing a first transition stimulus which returns the self-retaining suture 600 to the first temporary configuration shown in FIG. 6B. The first set of retainers 630 may then be elevated by providing a second transition stimulus which returns self-retaining suture 600 to the permanent configuration shown in FIG. 6A. The arrangement of the retainers need not be as shown in FIGS. 6A-6C. The retainers may be on different portions of filament 620 or the same portion of filament 620. The retainers 630, 632 may face in the same direction or different directions.

FIGS. 6D-6F illustrate an alternative programming for self-retaining suture 600. As shown FIG. 6D in this embodiment self-retaining suture 600 is formed in a permanent shape in which the retainers 630, 632 lay flat against filament 620. In the first temporary shape as shown in FIG. 6E, the retainers are elevated. In the second temporary shape, as shown in FIG. 6F, the retainers 630, 632 are positioned flat against filament 620. The self-retaining suture can then be deployed in either direction through tissue in the second temporary configuration. After deployment, a first transition stimulus is provided and the retainer 630, 632 assumes the first temporary shape in which retainers 630, 632 are elevated and engage tissue. In the first temporary shape, shown in FIG. 6E, retainers 630, 632 resist movement of the suture in both directions. If the second transition stimulus is provided, the retainers 630, 632 assume their permanent shape shown in FIG. 6D in which they lay flat against filament 620. Thus the second transition stimulus could be applied if it was desired to remove self-retaining suture 600.

### Composite Filaments Comprising Shape-Memory Materials

In alternative embodiments of the present invention retainers are formed in the surface of a composite shape-memory filament. In a composite shape memory filament, the filament includes two or more materials. At least one of the two or more materials is a shape memory material. It is advantageous to use composite shape-memory filaments to make self-retaining sutures. For example, by forming retainers in a composite shape-memory filament, a shape-memory self-retaining suture can be created in which the retainers elevate without requiring external mechanical elevation or in which the retainers self-elevate to augment the effects of mechanical elevation to produce a greater combined elevation. This is advantageous as it reduces the need for mechanical elevation which is time consuming, expensive and has the potential for weakening the retainers. Depending upon the combination of materials from which the composite shape-memory filament is made and the transition temperature of the shape memory component (or components), the retainers can be made to elevate prior to deployment, upon deployment into tissue, or after deployment into tissue.

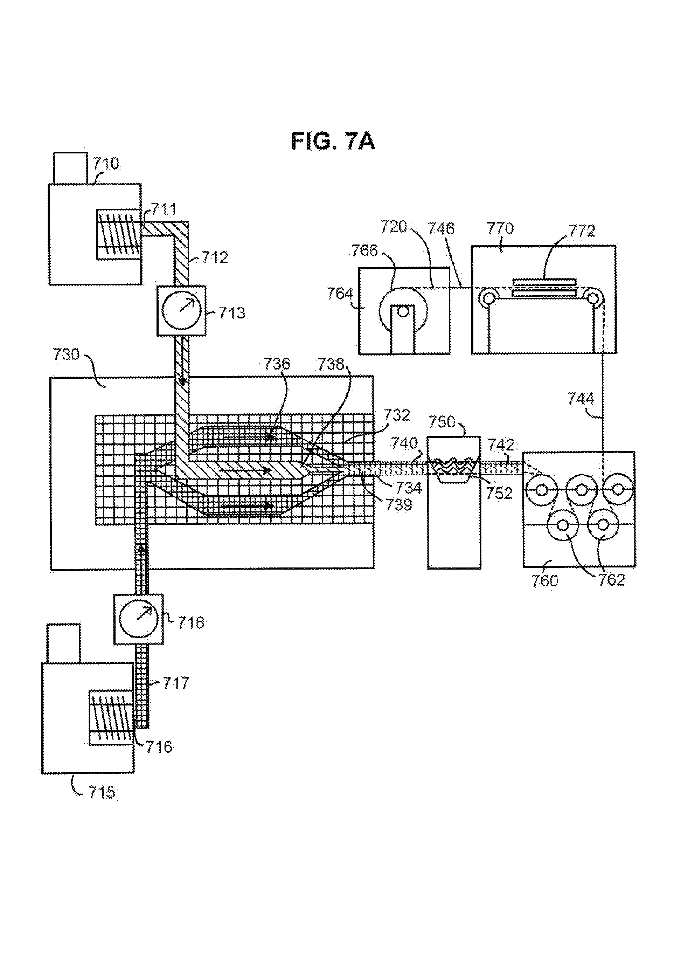

A composite filament can be made in many different ways. In accordance with one embodiment of the invention, a composite monofilament 720 is formed by co-extruding two materials. As shown in FIG.7A, satellite extruder 710 heats, melts and extrudes a first material 711 along conduit 712 to main extruder 730. Metering pump 713 on conduit 712 controls the flow of first material 711 to main extruder 730. A second satellite extruder 715 heats, melts and extrudes a second material 716 along conduit 717 to main extruder 730. Metering pump 718 on conduit 717 controls the flow of second material 716 to main extruder 730.

In main extruder 730, the two melted materials 711, 716 flow through two flow paths 736, 738 through an extrusion die 732 which controls the arrangement of the two materials 711, 716 when the materials combine in composite flow channel 739. The two materials are introduced into in composite flow channel 739 as shown and then extruded from die 732 through die exit 734. Die 732 and flow channels 736, 738, 739 are designed and operated such that the two materials 711 and 716 do not mix in composite flow channel 739. When making a composite shape-memory filament, at least one of the materials provided to the extrusion die is a shape-memory polymer.

The fiber 740 which is still melted material is then solidified by air or liquid cooling in quenching station 750. Quenching station 750 optionally includes a quenching bath 752 for liquid cooling. The solidified filament 742 is then drawn in drawing machine 760. Typically the solidified filament is drawn at temperatures between 70-80% of melting point (Celsius). Usually the suture is extruded then drawn on several rollers 762 with decreasing temperature. Drawing of the filament reduces the diameter of the filament while at the same time orienting the molecules of the polymers of the filament and enhancing the tensile strength of the filament. Typically drawing is conducted in a continuous process by winding the filament around a series of rollers 762 where each roller in the series has a slightly higher roller surface speed. The speed differential of the rollers results in stretching of the filament as the filament passes from roller to roller. The filament may also be tempered by one or more heating and cooling steps before, during or after the drawing process. As illustrated in FIG. 7A, drawn filament 744 is tempered in tempering machine 770 as the filament is passed through heating unit 772. After the filament has been drawn and tempered the finished monofilament 746 is passed to winder 764 where the composite monofilament 720 is wound onto drum 766 until required for preparation of self-retaining sutures.

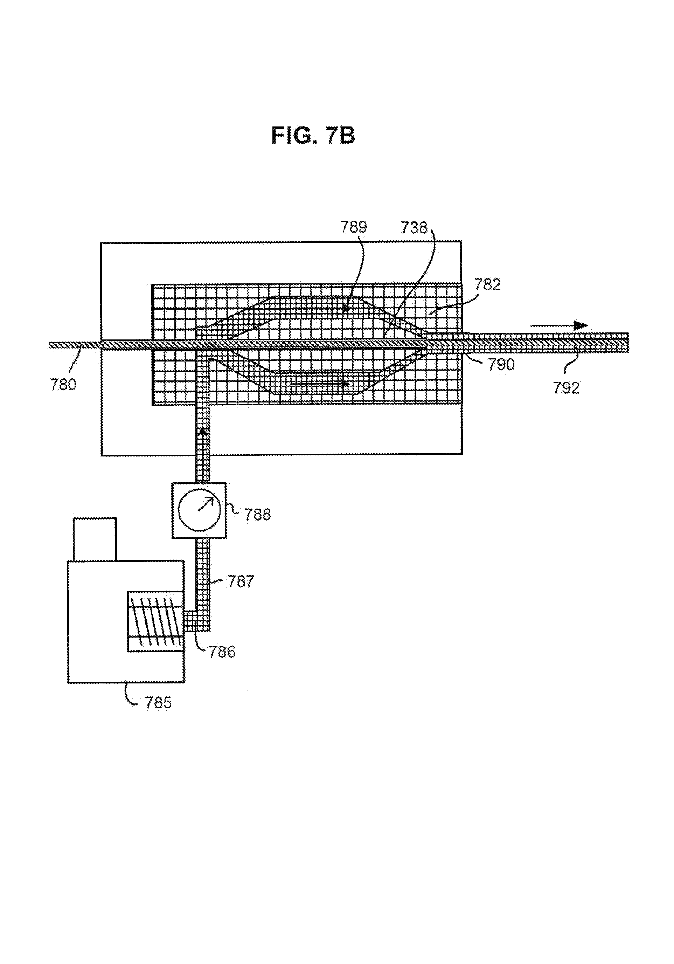

FIG. 7B illustrates an alternative method of making a filament suitable for use in embodiments of the present invention. As shown in FIG. 7B, a core filament 780 is drawn through an extrusion die 782. Satellite extruder 785 heats, melts and extrudes a sheath material 786 via conduit 787 to die 782. Metering pump 788 controls the flow of sheath material 786 to flow path 789 of die 782. The rate of supply of sheath material 786 and the rate of movement of core filament 780 are controlled such that a sheath material 786 is evenly coated on the core 780 in the desired cross-section determined by the cross-section of the extrusion nozzle 790. A suitable method for making a composite filament comprising a core coated with an extruded material is described in U.S. Patent 6,183,499 titled "Surgical Filament Construction" to Fisher et al. which is incorporated herein by reference. The finished filament 792 comprising core filament 780 and sheath material 786 may be quenched, tempered and drawn and then wound onto a drum as shown in FIG. 7A. However, in certain embodiments, core filament 780 may already have been drawn and no further drawing of finished filament 792 may be necessary or desirable. In some embodiments, for example, a core filament of a core material may be extruded and then drawn. Then the same material may be extruded over the core filament (as shown in FIG. 7B) without subsequent drawing of the filament. The resulting filament has a core and sheath of the same material, however, the sheath material has different physical properties than the core material because the sheath material has not undergone the drawing process.

The core filament may consist entirely of a non-shape-memory material, or may consist entirely of shape-memory material or may be a mixture of shape-memory materials and non-shape-memory materials. In one example, the core filament may comprise a braid of heterogeneous yarns wherein the heterogeneous yarns contain filaments made from a shape-memory alloy and non-biodegradable polymers. Exemplary yarns are disclosed in U.S. Patent Application Ser. No. 10/972,464 titled "Yarns Containing Filaments Made From Shape-memory Alloys".

Although extrusion has been illustrated in FIGS. 7A and 7B, any suitable manufacturing process may be used to form composite shape-memory filaments utilized for making self-retaining sutures according to the present invention. In an alternative embodiment, materials including at least one shape-memory material are spun into fibers to be used as monofilament or multifilament sutures. To produce fibers having a core/sheath structure, the core and sheath constituent materials are separately melted. The constituent materials are separately fed as polymer melts to a spinneret and are combined in the spinneret just before the spinneret exit orifice. The spinning device may have one or a plurality of spinnerets. The filament produced from a spinneret undergoes subsequent processing such as quenching, drawing and tempering in order to produce a composite filament suitable for use in embodiments of the present invention. Particular apparatus and methods for forming composite monofilaments suitable for use in the present invention can be found in U.S. Patent 7,070,610 titled "Monofilament Suture And Manufacturing Method Thereof' to Im et al. and U.S. Patent 6,315,788 titled "Composite Materials And Surgical Articles Made Therefrom" to Roby both of

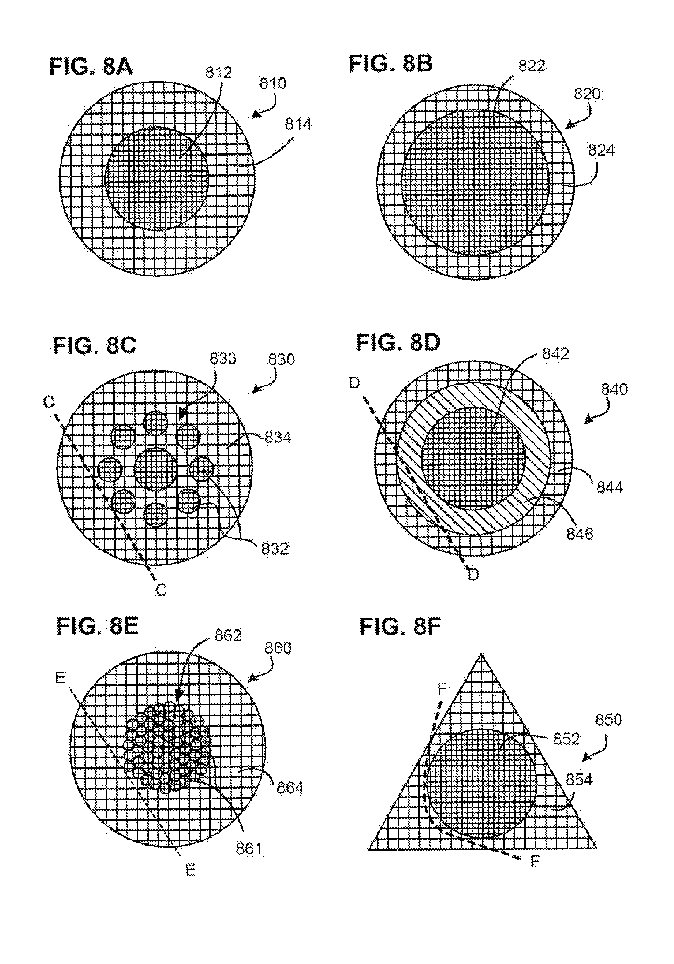

Depending upon the configuration of the extruders, die, spin block, spinneret, or other manufacturing equipment, a composite filament suitable for creating a self-retaining suture in accordance with embodiments of the present invention can be created with a wide variety of different arrangements of different materials. Furthermore, composite filaments can be made using 2, 3, 4 or even more different component materials if necessary or desired for the particular application. Examples of possible configurations of composite filaments are useful in specific embodiments of the present invention and are described below with respect to figures 8A-8F.

As shown in FIGS. 8A and 8B, simple composite filaments 810, 820 comprise two materials arranged one material in the core and a second material as a sheath over the core. This arrangement of materials in a composite filament can be made by co-extrusion of the two materials. In a simple variation, the two materials may be used in different amounts depending on the use to which the filament will be put. For example in FIG. 8A the core material 812 takes up about 25% of the cross-sectional area of filament 810, with the sheath material 814 taking up 75% of the cross-sectional area. In comparison, in FIG. 8B, the core material 822 and sheath material 824 each take up about 50% of the cross-sectional area. In general, the core material may comprise from 10% to 90% of the total cross-sectional area of the filament. Preferably the core material will comprise from 25% to 90% of the total cross-sectional area of the filament. More preferably the core material will comprise more than 50% of the total cross-sectional area of the filament.

The configuration of the particular materials in the composite filament will depend upon the characteristics of the materials and the amount of material necessary to fulfill the role of the filament. For example, in one embodiment sheath 814, may be made from a shape-memory polymer such that the elevation of retainers formed from the material of the sheath may be programmed. The depth of the sheath may be chosen such that the retainers when formed are formed entirely out of the sheath material. Alternatively, core 812 may be made from a shape memory material thus allowing the length of shape of the suture filament to be programmed. In some embodiments, different shape memory materials (having different transition stimuli) may be selected for core 812 and sheath 814 allowing retainer elevation and filament length and shape to be programmed and actuated somewhat independently.

FIG. 8C illustrates an alternative filament 830 in which a plurality of "islands" 832 are present in a surrounding "sea" 834 of the second material. The plurality of islands 832 together comprise a segmented core 833 of filament 830. The "sea" 834 of the second material comprises the sheath and also fills the interstices between the segments 832 of the segmented core 833. Either or both of the core and sheath may be formed from shape-memory polymer. In a typical embodiment of an self-retaining suture using filament 830, retainers are cut into the surface of filament 830 without penetrating to segmented core 833 as shown, for example by dashed line C-C. In this case, the retainers will be formed of the material of sheath 834 which may be a shape-memory polymer so that the elevation of retainers may be programmed. This arrangement of materials in a composite filament 830 can be made by co-extrusion of the two materials. The resulting fiber may show a useful combination of the characteristics of the materials. Particular configurations of composite monofilaments can be found in U.S. Patent 7,070,610 titled "Monofilament Suture And Manufacturing Method Thereof" to Im et al.

FIG. 8D illustrates another alternative composite filament 840 for use in the present invention. The composite filament of FIG. 8D is made from three different materials. A first material forms a core 842 of filament 840. A second material 844 forms a sheath on the outer surface of composite filament 842. The third material is sandwiched between the core 840 and the sheath 844 in intermediate layer 846. One, two or all of the core, intermediate layer and sheath may be formed from shape memory polymer. This arrangement of materials in a composite filament can be made by co-extrusion of the three materials. The material of intermediate layer 846 may be selected, for example, for its mechanical properties as an interface between the core 842 and sheath 844. Alternatively the material of intermediate layer 846 may be selected for favorable interaction with tissues in the retainers. In one embodiment of a self-retaining suture using filament 840, retainers are cut into the surface of filament 840 and into intermediate layer 846 without penetrating to core 842 as shown, for example by dashed line D-D. In this case, the retainers will be formed primarily of the material of sheath 834 which may be a shape-memory polymer so that the elevation of retainers may be programmed. Additionally, the material of intermediate layer 846 will be exposed to the tissue on the tissue retaining surface of the retainers where retainers are cut into filament 840. Thus, the material may be selected to promote engagement of the tissue and/or tissue healing. For example, the material of intermediate layer 846 may comprise an adhesive component, a therapeutic component or a material that promotes tissue adherence to the retainer or promotes wound healing.

FIG. 8E illustrates another alternative embodiment in which the core 862 of filament 860 comprises a plurality of filaments 861 braided together. Core 862 is surrounded by a sheath 864. This filament may be prepared by taking a braided thread (such as braided suture) and extruding the sheath onto the braided thread as it is passed through an extrusion die. Note that, as before, sheath 864 is sufficiently thick that creating retainers in the surface of filament 860 does not cut into core 862 or fibers 861 of core 862. For example, the maximum depth of a straight cut for a retainer is illustrated by dashed line E-E. Thus core 862 and the material of its fibers 861 may be engineered for high tensile strength and flexibility while sheath 864 is selected based upon it ability to form, elevate and deploy retainers. Either or both of the core and sheath may be formed from shape memory polymer. Core 862 may include some shape-memory fibers among other non-shape-memory fibers. The shape memory fibers may be made from shape-memory polymers or shape-memory metal alloys such as NITINOL. A method for making a composite filament comprising a braided core is described in U.S. Patent 6,183,499 titled "Surgical Filament Construction" to Fisher et al.

FIG. 8F illustrates an alternative embodiment in which the core and sheaths of filament 850 have different shapes. In the embodiment of FIG. 8F, core 852 has a circular cross-section while the sheath 854 has a triangular cross-section. This arrangement provides a greater volume of the second material at the apices of the triangle while still allowing the core material to provide a high percentage of the total cross-section of the filament. Either or both of the core and sheath may be formed from shape memory polymer. In this embodiment, the retainers are cut into the apices of the triangular cross section thus making optimal use of the material in the sheath 854. In addition, the retainer configuration may be selected such that retainers with arcuate bases are cut into the apices of the triangle. Dashed line F-F illustrates the cut for an arcuate base of a retainer and illustrates that the cut extends through a greater amount of the sheath 854 than would a straight cut. Methods for making self-retaining suture from filaments with triangular or other polyhedral cross-section are disclosed in U.S. Patent 5,342,376 titled "Inserting Device For A Barbed Tissue Connector" to Ruff which is incorporated herein by reference. The arrangement of materials in a composite filament shown in FIG. 8F can be made by co-extrusion of the two materials. The extruder nozzle is selected to have the desired shape. The shape of the cross-section of the filament matches the shape of the extruder nozzle. Alternatively, the filament may be formed as in FIG. 8A and then the sheath material 854 may be formed into the triangular shape by post-extrusion manipulations, such as using rollers to pinch the material into shape and then heating to anneal the polymer into the chosen shape prior to creation of the retainers. Naturally, other geometric arrangements of the sheath and core are possible, for example either the sheath or the core or both may be formed with a square cross-section, pentagonal, hexagonal or other polygonal cross-section.

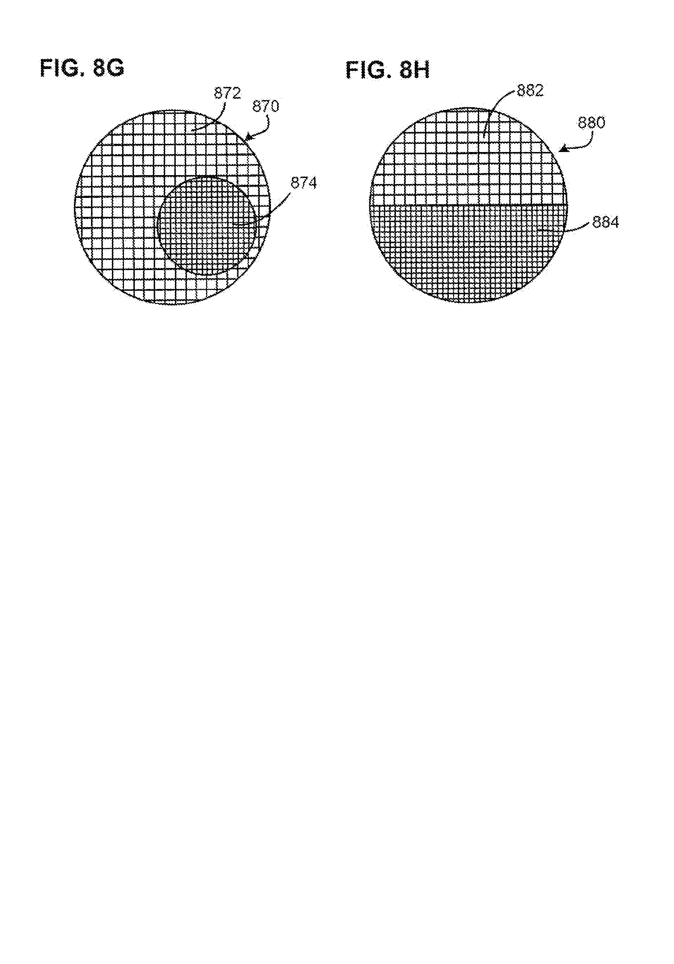

FIG. 8G shows an alternative embodiment of two materials in a filament 870. A sheath 872 covers a core 874 as in FIGS. 8A and 8B. However, in the filament of FIG. 8G, core 874 is eccentrically situated within the filament. FIG. 8H shows another alternative configuration in which a filament 880 has a lamellar arrangement of two materials. A first material 882 comprises one half of the filament and a second material 884 comprises the second half of the filament 880. Filament 880 also has an eccentric arrangement of the material 882, 884. Either or both of the materials in filaments 870, 880 may be formed from shape memory polymer. Where one of the materials is a shape-memory material configured to contract in response to a transition stimulus, the eccentric arrangement of the materials causes the filament to take on a spiral or helical structure with the contracted material on the inside of the spiral/helix. This spiral structure may provide a radius to assist elevation of retainers, it may also provide a semi-elastic shortening of the suture which may be beneficial in tissue elevation procedures.

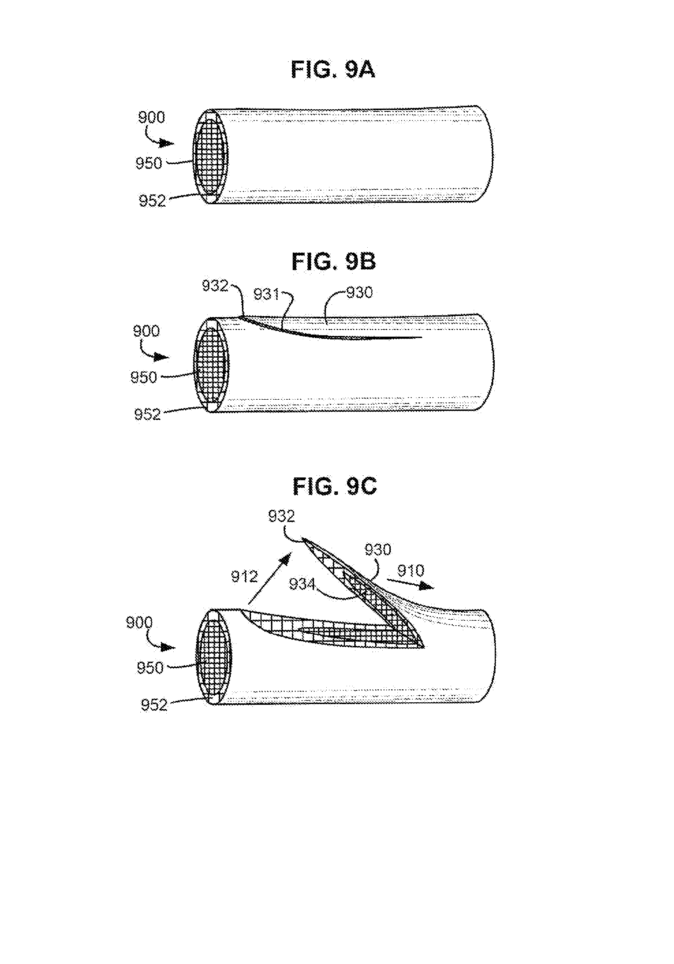

Referring now to FIG. 9A which shows a composite filament 900 which comprises a core 950 which is comprised of a polymer without shape-memory properties and a sheath 952 which is made out of a shape-memory polymer. The composite filament may be manufactured by extrusion of the two materials or by extrusion of the sheath over a preformed filament as described above with respect to FIGS. 7A and 7B or by other technology for coating a filament such as by dipping, deposition etc. In the example of FIG. 9A, sheath material 952 is stretched during the drawing process at a temperature above its transition temperature but below its melting temperature. Sheath material 952 is then cooled to a temperature below the transition temperature while still in the stretched configuration. Thus, sheath material 952 is in a temporary configuration and will return to a permanent shorter configuration upon exposure to the transition temperature. Core material 950 was also stretched during the drawing process, however core material 950 is not a shape memory polymer and will not shrink upon exposure to the transition temperature.

As shown in FIG. 9B, a retainer 930 may be formed in the surface of composite filament 900 by making a cut 931 into the surface of filament 900. As shown in FIG. 9B, retainer 930 is not elevated and tip 932 of retainer 930 is still flat against the surface of filament 900. However, when the transition stimulus is applied to filament 900 such as raising the temperature of filament 900 above the transition temperature, the shape-memory material of the sheath contracts. As shown in FIG. 9C when sheath 952 contracts in the direction of arrow 910, the tip 932 of retainer 930 is elevated above the surface of filament 900 as shown by arrow 912. The elevation of retainer tip 932 exposes inner retainer surface 934 and places filament 900 in a configuration in which retainer 930 can engage tissue.

This transition stimulus can be selected such that the sheath material shrinks when exposed to body temperature, moisture, pH or another natural chemical property of tissue to which the sheath material will only be exposed when deployed in the body. For example, polyether-ester shape-memory polymers can be activated to undergo contraction at transition temperatures near body temperature. Core material 950 is selected that does not shrink or shrinks less than the sheath material and is also selected to enhance the mechanical strength of the suture. Thus, when the suture is deployed into tissues of the body, sheath 952 contracts in the direction of arrows 910, and core 950 does not contract. Contraction of the sheath causes the retainers 930 to elevate from the position shown in FIG. 9B to the position shown in FIG. 9C in the direction of arrow 912. Thus, in this example, the retainers 930 elevate themselves without mechanical intervention after introducing the filament 900 into the tissues of a patient. This has the advantage that the suture can be inserted into the tissue in either direction prior to elevation of the retainers 930.

In alternative embodiments, an external stimulus may be required to cause elevation of the retainers. Such an external stimulus may be, for example, the application of heat to cause a temperature rise in the suture in excess of natural body temperature. The temperature rise can be caused by heating the suture outside the body prior to deployment. Alternatively, magnetic particles may be embedded in the material of the suture and caused to heat the suture material by magnetic induction caused by application of a magnetic field through the tissue of the subject after deployment of the suture. Additionally, shape-memory polymers which contract upon application of UV light, pH or other stimuli which may be applied to the suture after deployment in the tissues may be used in sheath 952. The external stimulus should be a stimulus that can reach and affect the suture without causing injury to tissue.

In alternative embodiments, retainers may be formed separately from a filament and then attached to a filament. Methods of manufacturing sutures with frusto-conical retainers have also been described, for example, in European Patent 1 075 843 and U.S. Pat. Publication No. 2007/0038429. Particular configurations of retainers are disclosed in United States Provisional Patent Application ANGIO-1016US0 entitled "Self-Retaining Sutures With BiDirectional Retainers Or Uni-Directional Retainers" to Goraltchouk et al. In accordance with embodiments of the present invention, either the filament or the retainers or both may comprise a shape-memory material such as a shape-memory polymer or shape-memory alloy. The shape memory components may be programmed/trained prior to assembly of the retainers and filament thereby allowing the use of shape-memory components having training/programming requirements which are incompatible with the other components of the self-retaining suture. For example, programming of a NITINOL alloy requires a temperature of several hundred degrees centigrade for several minutes. Many polymer filaments will melt or burn under these conditions. Thus, if NITINOL shape-memory components are to be combined with polymer components to make a shape-memory self-retaining suture it is preferable to train/program the NITINOL components prior to integration with the polymer components.

### Alternative Configurations

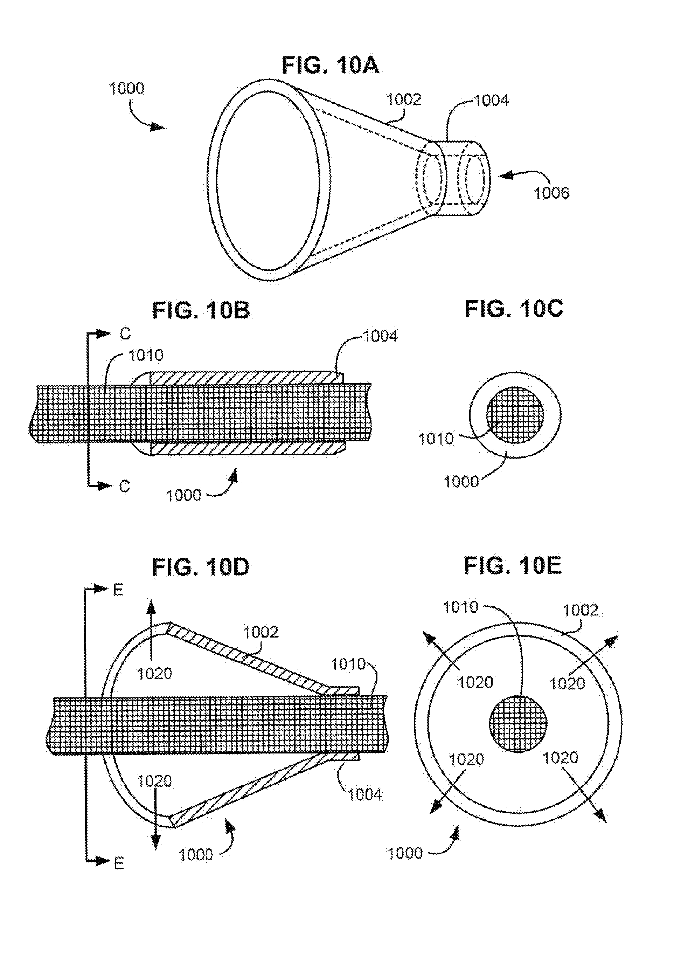

FIG. 10A illustrates an alternative configuration of shape-memory self-retaining suture in which a shape-memory retainer 1000 is formed separately and then added to a filament. As shown in FIG. 10A, retainer 1000 comprises a hollow cone 1002 and an eye 1004. Eye 1004 defines an aperture 1006 through which a filament may be threaded. FIG. 10A shows shape-memory retainer 1000 in its permanent programmed configuration. The shape of shape-memory retainer 1000 is trained/programmed in the standard manner appropriate for the material of which retainer 1000 is made. For example, if retainer 1000 is made of NITINOL, retainer 1000 may be formed in the shape shown in FIG. 10A or deformed into the shape shown in FIG. 10A and then annealed at high temperature while holding retainer 1000 in the shape shown in FIG. 10A.

As shown in FIG. 10B, after retainer 1000 is formed and/or programmed in the shape shown in FIG. 10A, retainer 1000 may be threaded over a filament 1010. Retainer 1000 may be held in place on filament 1010 by friction between eye 1004 and filament 1010. Alternatively, retainer 1000 may be secured in a position on filament 1010 using a knot, adhesive or other feature of filament 1010. After retainer 1000 has been positioned on filament 1010, retainer 1000 may be deformed to the temporary low profile configuration shown in FIG. 10B. FIG. 10C shows a partial sectional view of retainer 1000 along the line C-C of FIG. 10B. The combination of filament 1010 and a plurality of retainers 1000 creates a self-retaining suture thread. The self-retaining suture thread may be deployed through tissues in the low profile configuration shown in FIGS. 10B and 10C. Note that retainer 1000 may be deployed in either direction while in the temporary low profile configuration.

As shown in FIG. 10D, when a transition stimulus is applied to retainer 1000, such as by raising retainer 1000 to a transition temperature, retainer 1000 returns to the permanent configuration as shown in FIG. 10. The cone 1002 of retainer 1000 expands away from filament 1010 as shown by arrows 1020 in FIGS. 10D and 10E. FIG. 10E shows a partial sectional view of retainer 1000 along the line E-E of FIG. 10D. In the permanent configuration retainer 1000 resists motion of the filament 1010 and may be used to anchor filament 1010 in tissue. The transition stimulus may be selected such that retainer 1000 expands shortly after deployment in tissue. Alternatively, the transition stimulus may be selected such that an external transition stimulus is required to cause retainer 1000 to return to the permanent configuration of FIG. 10A.

As described above with respect to FIG. 4G and accompanying text, self-retaining suture systems may comprise more than two arms. A self-retaining suture system may have one, two or more arms including up to about ten arms or more depending upon the application and joined at a common connection. The connection may be a separate connector component or may be a point where the arms are fused, knotted, joined or welded together. A self-retaining suture system having more than two arms is useful in applications where it is desirable to have a plurality of suture lines radiating from a common point. Such self-retaining suture systems are useful for example in closes, puncture wounds, stellate wounds and other non-linear wounds. Such wounds can be produced by blunt trauma, gunshots, explosions and the like and are quite difficult to close with regular suturing techniques. FIGS. 10F-10H show some alternative embodiments of self-retaining suture systems having more than two arms.

Referring to FIG. 10F where a multi-arm self-retaining suture system 1030 is shown. As shown in FIG. 10F, self-retaining suture system 1030 comprises a connector 1031 comprising a ring 1032 having a number of channels or grooves 1033. Ring 1032 mates with a cap 1034. Cap 1034 has a number of clips 1035 which engage ring 1032 in a snap-fit. Connector 1031 may be made of the same materials described herein for the manufacture of sutures and may be made of an absorbable material or a non-absorbable material. In a simple embodiment, for example, the connector may be a shape-memory ring (such as a NITINOL ring) which contracts upon exposure to a transition stimulus thereby trapping the self-retailing suture threads passing through the ring without need for a cap.

As shown in FIG. 10F, two or more self-retaining sutures 1036 may be placed through ring 1032 such that the filament 1037 of each self-retaining suture 1036 passes through ring 1032 occupying a channel or groove 1033 in ring 1032. The physician selects the self-retaining suture 1036 based on the application and tissues to be connected and places the self-retaining sutures 1036 through ring 1032 as shown. Self-retaining sutures 1036 may comprise the same or different diameters and lengths of suture and the same or different needles. The physician then snaps cap 1034 into place in ring 1032 as shown by arrow 1038 until cap 1034 locks into place. Cap 1034 may be provided with a plurality of clips 1035 which engage ring 1032 and secure cap 1034 to ring 1032. When cap 1034 is locked into place, the suture threads of self-retaining suture systems 1036 are trapped between ring 1032 and cap 1034 where they pass through ring 1032. The cap thus locks self-retaining suture systems 1036 into place relative to connector 1031.

The connector may serve not only to connect the filaments of the self-retaining sutures but also to apply tension and reposition the self-retaining sutures. As shown in FIG. 10G, a multi-arm self-retaining suture system 1040 comprises a plurality of self-retaining suture threads 1042. Each self-retaining suture thread has an eye 1043 at one end and a needle 1044 at the other end. Five self-retaining suture threads 1042 are shown in FIG. 10G, however, the number of self-retaining suture threads may be between two and ten or more threads and is preferably between three and eight threads. A connector ring 1046 passes through each of the eyes 1043. The diameter of the connector ring 1046 may be reduced by pushing knot 1047 in the direction of arrow 1048. Knot 1047 serves to reduce the diameter of ring 1046 and then fix the diameter of ring 1046. Rather than a knotted filament, ring 1046 may comprise a filament and ratchet structure, or filament and locking pledget structure, or another structure for which the diameter can be controlled.

Reducing the diameter of ring 1046 draws each of the self-retaining suture threads 1042 in towards the center as shown by arrows 1049. The diameter of ring 1046 may be fixed prior to deploying self-retaining multi-arm self-retaining suture system 1040. However, in some applications it is desirable to deploy the threads of multi-arm self-retaining suture system 1040 into tissue with ring 1046 at a large diameter and then cause ring 1046 to contract drawing the tissues in which the threads 1042 are deployed towards each other in the direction of arrows 1049. The reduction in size of ring 1046 may be performed immediately after deployment of threads 1042 or at a later time, or in sequential steps as, for example, where the tissue stretches or swelling reduces over time allowing further approximation of the tissues toward the center point as shown.

FIG. 10H shows a multi-arm self-retaining suture system 1050 having three self-retaining suture threads 1051, 1052, 1053. Each of threads 1051, 1052, 1053 is joined at one end to a connector section 1058 and has a needle 1054, 1055, 1056 at the other end. Connector section 1058 is not a separate component in multi-arm self-retaining suture system 1050 but is a region where the material of self-retaining suture threads 1051, 1052, 1053 are joined to one another. The threads may be joined by melting, gluing welding or the like or may be formed in one piece. As shown in FIG. 10H, self-retaining suture thread 1051 is a larger diameter than self-retaining suture threads 1052 and 1053. Needle 1054 is accordingly larger than needles 1055, 1056. Needle 1054 is also a different configuration (curved) than needles 1055, 1056 (straight). FIG. 10H illustrates the wind range of variations that are possible in multi-arm self-retaining suture systems such as 1050. The arms of the suture system may be individually selected based upon the tissue in which they will be deployed. Not that the retainers on each arm of the suture are configured such that the retainers permit the suture thread of that arm to be deployed in the direction of the needle attached to that arm and resist movement of the suture thread in the direction towards the connector 1058. Thus, the suture threads may be deployed through tissue and the tissue approximated towards connector 1058, the retainers will then hold the tissue in the approximated position and resist movement of the tissue away from the connector 1058. Note that in some multi-arm systems it may be desirable to have some arms without retainers.

### Therapeutic Compounds And Materials

It is another advantage of the present invention that the sheath and/or outer layers of the filament may desirably incorporate materials that further promote tissue engagement. In addition to tissue engagement at the retainers, use of tissue engagement-promoting materials in at least part of the suture sheath surface (whether or not such materials also make up all or part of the retainers) can enhance the ability of the sutures to stay in place. One such class of tissue engagement-promoting materials are porous polymers that can be extruded, including both microporous polymers and polymers that can be extruded with bubbles (whether bioabsorbable or nonbioabsorbable). A suture synthesized with such materials in the sheath can have a three-dimensional lattice structure that increases tissue engagement surface area and permits tissue infiltration into the suture body itself, thus having a sheath structure that promotes successful suture use. Moreover, by optimizing pore size, fibroblast ingrowth can be encouraged, further facilitating the suture to be anchored in the tissue. One such microporous polymer is ePTFE (expanded polytetrafluoroethylene). Self-retaining incorporating ePTFE (and related microporous materials) in the sheath are well-suited to uses requiring a strong and permanent lift (such as breast lifts, face lifts, and other tissue repositioning procedures), as tissue infiltration of the suture results in improved fixation and engraftment of the suture and the surrounding tissue thus providing superior hold and greater longevity of the lift. Furthermore, an agent can be utilized in conjunction with the suture (introduced separately or adhered to the suture or incorporated into a material of the suture) to encourage fibrosis. Fibrosis-inducing agents which may be used in conjunction with a self-retaining suture in accordance with the present invention are described in U.S. Patent 7,166,570 titled "Medical Implants And Fibrosis-Inducing Agents" to Hunter et al.

Additionally, self-retaining sutures described herein may be provided with therapeutic compositions including, for example, compositions to promote healing and prevent undesirable effects such as scar formation, infection, pain, and so forth. This can be accomplished in a variety of manners, including for example: (a) by directly affixing to the suture a formulation (e.g., by either spraying the suture with a polymer/drug film, or by dipping the suture into a polymer/drug solution), (b) by coating the suture with a substance such as a hydrogel which will in turn absorb the composition, (c) by interweaving formulation-coated thread (or the polymer itself formed into a thread) into the suture structure in the case of multifilamentary sutures, (d) constructing the suture itself with a composition. Such compositions may include without limitation anti-proliferative agents, anti-thrombotic agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents. For example, a composition can be applied to the suture before the retainers are formed, so that when the retainers engage, the engaging surface is substantially free of the coating. In this way, tissue being sutured contacts a coated surface of the suture as the suture is introduced, but when the retainer engages, a non-coated surface of the retainer contacts the tissue. Alternatively, the suture may be coated after or during formation of retainers on the suture if, for example, a fully-coated rather than selectively-coated suture is desired. In yet another alternative, a suture may be selectively coated either during or after formation of retainers by exposing only selected portions of the suture to the coating. The particular purpose to which the suture is to be put or the composition may determine whether a fully-coated or selectively-coated suture is appropriate; for example, with lubricious coatings, it may be desirable to selectively coat the suture, leaving, for instance, the tissue-engaging surfaces of the sutures uncoated in order to prevent the tissue engagement function of those surfaces from being impaired. On the other hand, coatings such as those comprising such compounds as anti-infective agents may suitably be applied to the entire suture, while coatings such as those comprising fibrosing agents may suitably be applied to all or part of the suture (such as the tissue-engaging surfaces). The purpose of the suture may also determine the sort of coating that is applied to the suture; for example, self-retaining sutures having anti-proliferative coatings may be used in closing tumor excision sites, while self-retaining sutures with fibrosing coatings may be used in tissue repositioning procedures and those having anti-scarring coatings may be used for wound closure on the skin. As well, the structure of the suture may influence the choice and extent of coating; for example, sutures having an expanded segment may include a fibrosis-inducing composition on the expanded segment to further secure the segment in position in the tissue. Coatings may also include a plurality of compositions either together or on different portions of the suture, where the multiple compositions can be selected either for different purposes (such as combinations of analgesics, anti-infective and anti-scarring agents) or for the synergistic effects of the combination.

In accordance with embodiments of the present invention, the shape memory properties of the suture may be utilized to influence where, when and/or how the therapeutic compounds or materials are exposed to tissue and/or released into the tissue. First, in certain embodiments, self-retaining sutures may be made in which the therapeutic compound or agent is only exposed upon elevation of the retainer. As discussed above, shape-memory materials can be used to make retainers that may be elevated or made to lay flat against the filament in response to a transition stimulus. Thus, for example, where the therapeutic agent is only exposed to tissue on the tissue retainer surface, the therapeutic agent may be protected during insertion of the self-retaining suture into tissue and then the retainer may be elevated by application of a transition stimulus, thereby exposing the therapeutic agent to the tissue.

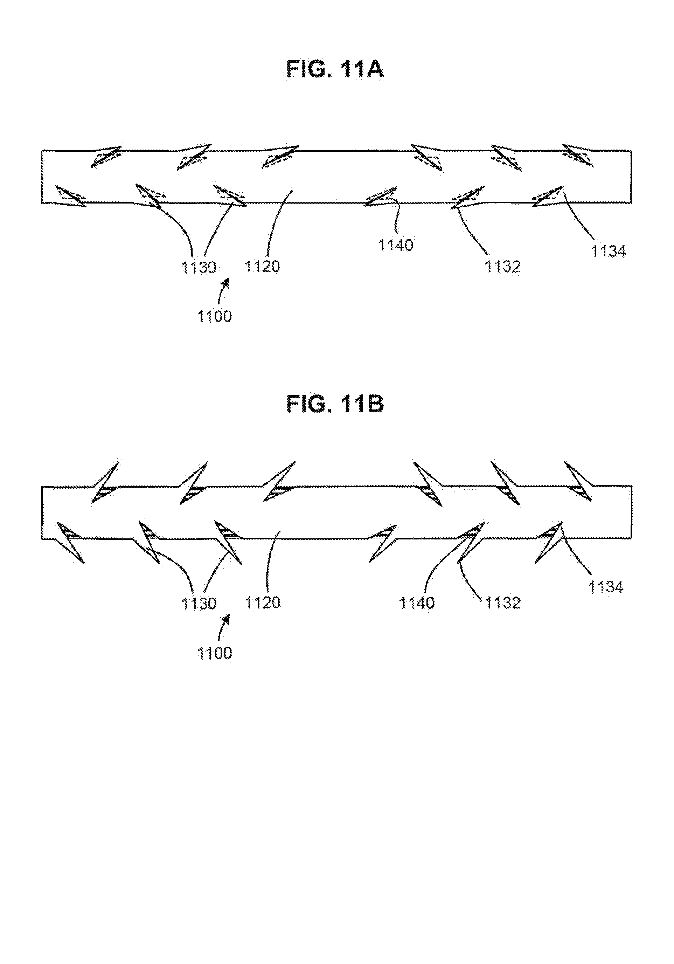

FIG. 11A shows a shape-memory self-retaining suture 1100 having a plurality of retainers 1130 on a filament 1120. As shown in FIG. 11A the retainers 1130 are not elevated and the tips 1132 of retainers 1130 are adjacent the surface of filament 1120. At the base 1134 of each retainer 1130 is a region 1140 comprising a therapeutic agent. The regions 1140 may be created by selective application of the therapeutic agent to the shape-memory self-retaining suture 1100 after formation of the retainers 1130. Alternatively, filament 1120 may comprise the therapeutic agent as an inner material that is exposed by cutting retainers 1130 into the surface of filament 1120. When retainers 1130 are in the non-elevated position, the regions 1140 are covered by the retainers 1130 and the therapeutic agent is not exposed on the surface of filament.

As shown in FIG. 11B, upon application of a transition stimulus to the shape-memory self-retaining suture 1100 the retainers 1130 are caused to elevate and the tips 1132 of retainers 1130 move away from the surface of filament 1120 into a position ready to engage tissue. When the transition stimulus is applied and the retainers are elevated regions 1140 of therapeutic agent are exposed. Thus, the therapeutic agent is exposed to tissue and/or released into the tissue by application of the transition stimulus to elevate the retainers. In alternative embodiments, the release of the therapeutic agent may be controlled using a different transition stimulus than used for elevation of the retainers. Thus, the timing and location of the release of the therapeutic agent may be controlled by selective application of the transition stimulus to the shape-memory self-retaining suture.

### Clinical Uses

In addition to the general wound closure and soft tissue repair applications, self-retaining sutures can be used in a variety of other indications.

Self-retaining sutures described herein may be used in various dental procedures, i.e., oral and maxillofacial surgical procedures and thus may be referred to as "self-retaining dental sutures." The above-mentioned procedures include, but are not limited to, oral surgery (e.g., removal of impacted or broken teeth), surgery to provide bone augmentation, surgery to repair dentofacial deformities, repair following trauma (e.g., facial bone fractures and injuries), surgical treatment of odontogenic and non-odontogenic tumors, reconstructive surgeries, repair of cleft lip or cleft palate, congenital craniofacial deformities, and esthetic facial surgery. Self-retaining dental sutures may be degradable or non-degradable, and may typically range in size from USP 2-0 to USP 6-0.

Self-retaining sutures described herein may also be used in tissue repositioning surgical procedures and thus may be referred to as "self-retaining tissue-repositioning sutures". Such surgical procedures include, without limitation, face lifts, neck lifts, brow lifts, thigh lifts, and breast lifts. Self-retaining sutures used in tissue repositioning procedures may vary depending on the tissue being repositioned; for example, sutures with larger and further spaced-apart retainers may be suitably employed with relatively soft tissues such as fatty tissues. In particular, shape-memory self-retaining sutures may be employed where the shape-memory suture is programmed to shrink in response to the application of a transition stimulus thereby repositioning the tissue in which it is implanted. Thus a self-retaining suture may be anchored in tissue to a stable anatomical feature. Applying a transition stimulus selectively to the self-retaining suture would then lift the tissue towards the stable anatomical feature. The amount of lift could be selected by selective application of the transition stimulus to different regions of the suture.

Self-retaining sutures described herein may also be used in microsurgical procedures that are performed under a surgical microscope (and thus may be referred to as "self-retaining microsutures"). Such surgical procedures include, but are not limited to, reattachment and repair of peripheral nerves, spinal microsurgery, microsurgery of the hand, various plastic microsurgical procedures (e.g., facial reconstruction), microsurgery of the male or female reproductive systems, and various types of reconstructive microsurgery. Microsurgical reconstruction is used for complex reconstructive surgery problems when other options such as primary closure, healing by secondary intention, skin grafting, local flap transfer, and distant flap transfer are not adequate. Self-retaining microsutures have a very small caliber, often as small as USP 9-0 or USP 10-0, and may have an attached needle of corresponding size. The microsutures may be degradable or non-degradable.

Self-retaining sutures as described herein may be used in similarly small caliber ranges for ophthalmic surgical procedures and thus may be referred to as "ophthalmic self-retaining sutures". Such procedures include but are not limited to keratoplasty, cataract, and vitreous retinal microsurgical procedures. Ophthalmic self-retaining sutures may be degradable or non-degradable, and have an attached needle of correspondingly-small caliber.

Self-retaining sutures can be used in a variety of veterinary applications for a wide number of surgical and traumatic purposes in animal health.

## Claims

1. A self-retaining suture comprising: a filament having a length;
a plurality of retainers disposed on the filament, each retainer having a retainer base engaging the filament; and a first shape-memory material;
wherein the self-retaining suture has a first form and a second form, and wherein a transition in the suture from the first form to the second form is effectuated by exposure to an electromagnetic stimulus;
**characterized in that** the electromagnetic stimulus is selected from the group consisting of UV radiation, IR radiation, visible light, and laser light, and wherein the source of the stimulus is connectable to an end of the filament such that said stimulus travels through said filament.

2. The suture of claim 1, wherein the first form comprises elevated retainers, or wherein the first form comprises an increased filament length, or wherein the second form comprises elevated retainers, or wherein the second form comprises an increased filament length, or wherein at least one of the filament and the retainers comprises the shape-memory material.

3. The suture of claim 1, further comprising a third form, wherein a transition in the suture from the second form to the third form is effectuated by a secondary transition stimulus, wherein the third form comprises elevated retainers, or wherein the third form comprises an increased filament length, and wherein the secondary transition stimulus is selected from amongst:
a) a selected electric field
b) a selected magnetic field
c) electromagnetic radiation of a selected magnitude
d) a selected temperature
e) a selected pressure
f) a selected pH
g) a selected chemical environment
h) a selected solvation environment

4. The suture of claim 1, wherein the shape-memory material is selected from the class comprised of polymers, thermoplastics, metal alloys, hydrogels and ceramics, and wherein the metal alloy is a nickel-titanium alloy.

5. The suture of claim 1, comprising a first material and a second material, and wherein the first material is the first shape-memory material, and wherein the retainers comprise at least in part the first shape-memory material, and wherein the suture comprises at least in part the first shape-memory material, and wherein the second material is a second shape-memory material, and wherein the retainers comprise at least in part the second shape-memory material and wherein the suture comprises at least in part the second shape-memory material.

6. A self-retaining suture system, comprising at least one suture as claimed in claim 1.

## Patentansprüche

1. Selbst-rückhaltendes Nahtmaterial umfassend: einen Faden mit einer Länge; eine Vielzahl von Rückhalteelementen, die an dem Faden angeordnet sind, wobei jedes Rückhalteelement eine Rückhalteelementbasis aufweist, die den Faden fasst; und ein erstes Formgedächtnismaterial;
wobei das selbst-rückhaltende Nahtmaterial eine erste Form und eine zweite Form aufweist und wobei ein Übergang in dem Nahtmaterial von der ersten Form zu der zweiten Form durch Exposition gegenüber einem elektromagnetischen Reiz bewirkt wird;
**dadurch gekennzeichnet, dass** der elektromagnetische Reiz ausgewählt ist aus der Gruppe bestehend aus UV-Strahlung, IR-Strahlung,
sichtbarem Licht und Laserlicht und wobei die Quelle des Reizes mit einem Ende des Fadens verbindbar ist, so dass der Reiz durch den Faden wandert.

2. Nahtmaterial gemäß Anspruch 1, wobei die erste Form hochgestellte Rückhalteelemente umfasst oder wobei die erste Form eine vergrößerte Fadenlänge umfasst oder wobei die zweite Form hochgestellte Rückhalteelemente umfasst oder wobei die zweite Form eine vergrößerte Fadenlänge umfasst oder wobei wenigstens eines von dem Faden und den Rückhalteelementen das Formgedächtnismaterial umfasst.

3. Nahtmaterial gemäß Anspruch 1, ferner umfassend eine dritte Form, wobei ein Übergang in dem Nahtmaterial von der zweiten Form zu der dritten Form durch einen sekundären Übergangsreiz bewirkt wird, wobei die dritte Form hochgestellte Rückhalteelemente umfasst oder wobei die dritte Form eine vergrößerte Fadenlänge umfasst und wobei der sekundäre Übergangsreiz ausgewählt ist aus:
a) einem ausgewählten elektrischen Feld
b) einem ausgewählten Magnetfeld
c) elektromagnetischer Strahlung mit einer ausgewählten Stärke
d) einer ausgewählten Temperatur
e) einem ausgewählten Druck
f) einem ausgewählten pH-Wert
g) einer ausgewählten chemischen Umgebung
h) einer ausgewählten Lösungsumgebung.

4. Nahtmaterial gemäß Anspruch 1, wobei das Formgedächtnismaterial ausgewählt ist aus der Klasse bestehend aus Polymeren, Thermoplasten, Metalllegierungen, Hydrogelen und Keramik und wobei die Metalllegierung eine Nickel-Titan-Legierung ist.

5. Nahtmaterial gemäß Anspruch 1, umfassend ein erstes Material und ein zweites Material, wobei das erste Material das erste Formgedächtnismaterial ist und wobei die Rückhalteelemente wenigstens teilweise das erste Formgedächtnismaterial umfassen und wobei das Nahtmaterial wenigstens teilweise das erste Formgedächtnismaterial umfasst und wobei das zweite Material ein zweites Formgedächtnismaterial ist und wobei die Rückhalteelemente wenigstens teilweise das zweite Formgedächtnismaterial umfassen und wobei das Nahtmaterial wenigstens teilweise das zweite Formgedächtnismaterial umfasst.

6. Selbst-rückhaltendes Nahtmaterialsystem, umfassend wenigstens ein Nahtmaterial gemäß Anspruch 1.

## Revendications

1. Suture auto-rétentive comprenant:
un filament présentant une certaine longueur;
une pluralité d'éléments de retenue disposés sur le filament, chaque élément de retenue présentant une base d'élément de retenue qui engage le filament; et
et un premier matériau à mémoire de forme,
dans laquelle la suture auto-rétentive présente une première forme et une deuxième forme; et
dans laquelle une transition dans la suture entre la première forme et la deuxième forme est effectuée par une exposition à un stimulus électromagnétique,
**caractérisée en ce que** le stimulus électromagnétique est sélectionné dans le groupe comprenant un rayonnement ultraviolet, un rayonnement infrarouge, une lumière visible et une lumière laser, et dans laquelle la source du stimulus peut être connectée à une extrémité du filament, de telle sorte que ledit stimulus voyage à travers ledit filament.

2. Suture selon la revendication 1, dans laquelle la première forme comprend des éléments de retenue élevés, ou dans laquelle la première forme comprend une longueur de filament accrue, ou dans laquelle la deuxième forme comprend des éléments de retenue élevés, ou dans laquelle la deuxième forme comprend une longueur de filament accrue, ou dans laquelle au moins un parmi le filament et les éléments de retenue comprend le matériau à mémoire de forme.

3. Suture selon la revendication 1, comprenant en outre une troisième forme, dans laquelle une transition dans la suture entre la deuxième forme et la troisième forme est effectuée par un stimulus de transition secondaire, dans laquelle la troisième forme comprend des éléments de retenue élevés, ou dans laquelle la troisième forme comprend une longueur de filament accrue, et dans laquelle le stimulus de transition secondaire est sélectionné parmi:
a) un champ électrique sélectionné
b) un champ magnétique sélectionné
c) un rayonnement électromagnétique d'une grandeur sélectionnée
d) une température sélectionnée
e) une pression sélectionnée
f) un pH sélectionné
g) un environnement chimique sélectionné
h) un environnement de solvatation sélectionné

4. Suture selon la revendication 1, dans laquelle le matériau à mémoire de forme est sélectionné dans la classe constituée de polymères, de thermoplastiques, d'alliages de métal, d'hydrogels et de céramiques, et dans laquelle l'alliage de métal est un alliage de nickel et de titane.

5. Suture selon la revendication 1, comprenant un premier matériau et un second matériau, et dans laquelle le premier matériau est le premier matériau à mémoire de forme, et dans laquelle les éléments de retenue comprennent au moins en partie le premier matériau à mémoire de forme, et dans laquelle la suture comprend au moins en partie le premier matériau à mémoire de forme, et dans laquelle le second matériau est un second matériau à mémoire de forme, et dans laquelle les éléments de retenue comprennent au moins en partie le deuxième matériau à mémoire de forme, et dans laquelle la suture comprend au moins en partie le second matériau à mémoire de forme.

6. Système de suture auto-rétentive, comprenant au moins une suture selon la revendication 1.
